# EUROPEAN PATENT APPLICATION

(11) **EP 3 112 474 A1**
(43) Date of publication of application: **04.01.2017**
(21) Application number: 15174270.7
(22) Date of filing: 29.06.2015
(51) Int. Cl.: C12Q 1/68

(54) **METHOD OF DETECTING AVIAN NECROTIC ENTERITIS**

(71) Applicant: Evonik Degussa GmbH, 45128 Essen (DE)
(72) Inventor: IGWE, Emeka-Ignatius, 80333 München (DE); WALLMEIER, Holger, 65843 Sulzbach/Ts. (DE); PELZER, Stefan, 33335 Gütersloh (DE); FLÜGEL, Monika, 33803 Steinhagen (DE); BEKEL, Thomas, 33790 Halle (Westf.) (DE)

(57) **Abstract**

The current invention relates to a microvesicle based method of detecting avian necrotic enteritis as well as to new markers which have been identified as suitable for detection of avian necrotic enteritis and/or avian infection by Clostridium perfringens:

## Description

The current invention relates to a microvesicle based method of detecting avian necrotic enteritis as well as to new markers which have been identified as suitable for detection of avian necrotic enteritis and/or avian infection involving Clostridium perfringens.

Clostridium perfringens is the main causative agent of avian necrotic enteritis (NE), an enteric disease of poultry that was first described in 1961. NE in chickens manifests as an acute or chronic enterotoxemia. The acute disease results in significant levels of mortality due to the development of necrotic lesions in the gut wall, whereas the chronic disease leads to a significant loss of productivity and welfare. It has been estimated that the disease results in damages of several billion US-Dollars per year for the poultry industry.

C. perfringens is commonly found in the gastrointestinal tract of poultry, the occurrence of necrotic enteritis, however, is sporadic. C. perfringens is a Gram-positive, rod-shaped, spore forming, oxygen-tolerant anaerobe. C. perfringes strains are classified into five toxin types (A, B, C, D and E), based on the production of four suspected major toxins (alpha, beta, epsilon and iota). While type A is consistently recovered from intestines of chicken, the other types are less common.

Early studies on NE suggested that the main virulence factor involved in the disease was secreted by the bacteria, which led to the proposal that a phospholipase C enzyme, called alpha-toxin, was the major toxin involved in pathogenesis. But recent studies showed that alpha-toxin seems not to be an essential virulence factor since alpha toxin mutant strains were capable of causing NE, which questions the role of alpha-toxin in the disease in general. In more recent studies, the novel pore forming toxin, netB, has been suggested to play a major key role in the development of this disease.

Apart from the mere association of the virulence factors per se to incidence of NE, there is still a great burden to show the mechanistic link between these factors to the establishment of the disease in birds or farm animals. Moreover, relating subclinical NE infection to any of these virulence factors is extremely difficult to achieve since birds are void of signs to warrant their examination earlier before slaughter. Therefore a need for an early method of detecting NE, and most importantly, the subclinical forms of NE remains imperative.

Traditionally, the discovery and validation of biomarkers for disease requires the use of tissue, either fresh frozen, or more commonly, formalin-fixed, paraffin-embedded. This has been especially true for mRNA based biomarkers, because RNase degradation of free RNA makes translation of tissue based biomarkers into biofluids such as blood or fecal/cecal samples highly problematic. But monitoring of tissue biomarkers is thus, because of its invasive nature, time-consuming and impractical, when large numbers of samples are involved like for farm animals.

Non-invasisve methods such as the measurement of blood parameters have been described for the detection of diseases in birds (Chuku et al., 2012; Aade et al., 2012; Saleem, 2012). Gulbeena Saleem (2012) suggests as an alternative approach for the determination of sub-clinical NE the measurement of the levels of acute phase proteins like ceruplasmin, PIT54 and ovotransferrin in response to C. perfringes challenge in blood serum of the chickens. It turned out that only ceruplasmin seemed to be a suitable acute phase protein correlating with the occurrence of sub-clinical NE. Notwithstanding, this can be seen as a problem, since ceruplasmin is not a specific marker for NE but for diseases caused by other bacterial pathogens (collibacillosis in chicken, Piercy, 1979; salmonella infection in commercial layers, Garcia *et al.,* 2009), as well.

However, it was recently discovered that exosomes and other membrane vesicles found in biomaterials contain stable RNA from human donor cells, including mRNA, miRNA and other non-coding RNA with high specificity to disease conditions (Skog et al., 2008; Nilsson et al., 2009; Li et al., 2009; Shao et al., 2012).

Thus the inventors of the current application wondered, if membrane vesicles isolated from bodily fluids or excrements of poultry might be used to determine poultry exhibiting sub-clinical necrotic enteritis. This might be in particular true due to an accumulation of specific host miRNAs showing correlation to the disease.

Surprisingly it was found that microvesicles isolated from avian bodily fluids or avian excrements did exhibit an alteration in the content of detectable RNA in dependence of the presence of necrotic enteritis. Besides an increase of specific host-miRNAs in the microvesicles due to infection by C. perfringens, surprisingly also mRNA sequences of C. perfringens could be detected in the microvesicle fraction, which are therefore classified as disease specific virulence factors and accordingly as suitable biomarkers indicative for necrotic enteritis.

The occurrence of C. perfringens mRNA in the microvesicle fraction might be explained by the fact that the isolation procedure applied does not only lead to an enrichment of microvesicles of poultry, but also to an enrichment of microvesicles of the infectious bacterium. Surprisingly, besides known putative virulence factors like netB, also previously non-suspective RNA sequences could be identified as being correlative to necrotic enteritis.

It was thus a first object of the current invention to provide a fast and reliable, preferably non-invasive, method for determining avian necrotic enterititis, in particular sub-clinical avian necrotic enterititis, preferably in poultry, more preferably in chicken and/or to identify avian subjects which suffer from necrotic enteritis.

It was a further object of the current invention to identify suitable markers, preferably disease-specific markers, which can be employed in such a method.

Thus a first subject of the current invention is a method of detecting avian necrotic enteritis, in particular sub-clinical avian necrotic enteritis, the method comprising isolating microvesicles from an avian sample, in particular from an avian bodily fluid or an avian excrement, and subsequently determining the presence and/or the level of at least one marker indicative for necrotic enteritis in these microvesicles, wherein the presence and/or an increased level of this at least one marker in comparison to a non-infected control is indicative for necrotic enteritis.

The marker indicative for necrotic enteritis according to the invention is preferably selected from the following sequences of Clostridium perfringens and homologues and fragments thereof: the SAM region (SEQ ID NO: 1), the swim zinc finger region (SEQ ID NO: 3), the scRNA (SEQ ID NO: 4), the colA region (SEQ ID NO: 5), the CPE0956 region (SEQ ID NO: 7), the netB region (SEQ ID NO: 8), the virX region (SEQ ID NO: 9) and the TpeL region (SEQ ID NO: 16); as well as from the following avian sequences and homologues and fragments thereof: mir-16 (SEQ ID NO: 10), mir-24 (SEQ ID NO: 11), mir-155 (SEQ ID NO: 12), mir-206 (SEQ ID NO: 13).

Thus a preferred subject of the current invention is a method of detecting avian necrotic enteritis, in particular sub-clinical avian necrotic enteritis, the method comprising isolating microvesicles from an avian sample, in particular from avian bodily fluids or avian excrements, and subsequently determining the level of at least one marker indicative for necrotic enteritis in these microvesicles, wherein an increased level of this at least one marker in comparison to a non-infected control is indicative for necrotic enteritis and wherein the marker is selected from the following group ("List I"):
a) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 1;
b) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 3;
c) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 4;
d) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 5;
e) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 7;
f) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 8;
g) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 9;
h) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 16;
i) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40, 60 or 80, consecutive nucleotides of the nucleotide sequence as depicted in SEQ ID NO: 10;
j) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 11;
k) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 12;
l) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 13;
m) polynucleotides (DNAs and RNAs) which are complementary to the sequences according to (a) to (I);
n) polynucleotides (DNAs and RNAs) which are complementary to the sequences according to (m);
o) polynucleotides which comprise the polynucleotides according to (a) to (n).

In this method the sequences are preferably selected from the following group ("List II"):
a) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200 consecutive nucleotides of the nucleotide sequence according to position 1 to 630, in particular 420 to 630, preferably 460 to 560, of SEQ ID NO: 1,
b) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence according to position 600 to 1000, in particular 670 to 760, of SEQ ID NO: 3,
c) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence according to position 60 to 240, in particular 80 to 220, of SEQ ID NO: 4;
d) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides according to position 1300 to 1800, in particular 1400 to 1700, of (the sequence as depicted in) SEQ ID NO: 5;
e) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence according to position 1 to 591, in particular 100 to 280 of SEQ ID NO: 7;
f) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence according to position 240 to 540, in particular 300 to 540 of SEQ ID NO: 8;
g) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence according to position 12 to 418, in particular 240 to 400, of SEQ ID NO: 9;
h) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence according to position 673 to 5628 of SEQ ID NO: 16;
i) polynucleotides which have a sequence identity of at least 80 %, preferably at least 85, 90 or 95 %, most preferably 100 %, to a polynucleotide which comprises at least 8 or 10, preferably at least 12, consecutive nucleotides of the nucleotide sequence according to position 14 to 25 of SEQ ID NO: 10;
j) polynucleotides which have a sequence identity of at least 80 %, preferably at least 85, 90 or 95 %, most preferably 100 %, to a polynucleotide which comprises at least 8 or 10, preferably at least 12, 14, 16, 18 or 20, consecutive nucleotides of the nucleotide sequence according to position 44 to 65 of SEQ ID NO: 11;
k) polynucleotides which have a sequence identity of at least 80 %, preferably at least 85, 90 or 95 %, most preferably 100 %, to a polynucleotide which comprises at least 8 or 10, preferably at least 12, 14, 16, 18 or 20, consecutive nucleotides of the nucleotide sequence according to position 3 to 24 of SEQ ID NO: 12;
l) polynucleotides which have a sequence identity of at least 80 %, preferably at least 85, 90 or 95 %, most preferably 100 %, to a polynucleotide which comprises at least 8 or 10, preferably at least 12, 14, 16, 18 or 20, consecutive nucleotides of the nucleotide sequence according to position 45 to 66 of SEQ ID NO: 13,
m) polynucleotides (DNAs and RNAs) which are complementary to the sequences according to (a) to (I);
n) polynucleotides (DNAs and RNAs) which are complementary to the sequences according to (m);
o) polynucleotides which comprise the polynucleotides according to (a) to (n).

More preferably the sequences are selected from the following group ("List III"):
a) polynucleotides which have a sequence identity of at least 80 %, preferably at least 85, 90 or 95 %, most preferably 100 %, to a polynucleotide which comprises at least 20, preferably at least 30 or 40, consecutive nucleotides of the nucleotide sequence according to position 460 to 560 of SEQ ID NO: 1,
b) polynucleotides which have a sequence identity of at least 80 %, preferably at least 85, 90 or 95 %, most preferably 100 %, to a polynucleotide which comprises at least 20, preferably at least 30 or 40, consecutive nucleotides of the nucleotide sequence according to position 670 to 760 of SEQ ID NO: 3,
c) polynucleotides which have a sequence identity of at least 80 %, preferably at least 85, 90 or 95 %, most preferably 100 %, to a polynucleotide which comprises at least 20, preferably at least 30 or 40, consecutive nucleotides of the nucleotide sequence according to position 80 to 220 of SEQ ID NO: 4;
d) polynucleotides which have a sequence identity of at least 80 %, preferably at least 85, 90 or 95 %, most preferably 100 %, to a polynucleotide which comprises at least 20, preferably at least 30 or 40, consecutive nucleotides of the nucleotide sequence according to position 1400 to 1700 of SEQ ID NO: 5;
e) polynucleotides which have a sequence identity of at least 80 %, preferably at least 85, 90 or 95 %, most preferably 100 %, to a polynucleotide which comprises at least 20, preferably at least 30 or 40, consecutive nucleotides of the nucleotide sequence according to position 100 to 280 of SEQ ID NO: 7;
f) polynucleotides which have a sequence identity of at least 80 %, preferably at least 85, 90 or 95 %, most preferably 100 %, to a polynucleotide which comprises at least 20, preferably at least 30 or 40, consecutive nucleotides of the nucleotide sequence according to position 300 to 540 of SEQ ID NO: 8;
g) polynucleotides which have a sequence identity of at least 80 %, preferably at least 85, 90 or 95 %, most preferably 100 %, to a polynucleotide which comprises at least 20, preferably at least 30 or 40, consecutive nucleotides of the nucleotide sequence according to position 240 to 400 of SEQ ID NO: 9;
h) polynucleotides which have a sequence identity of at least 80 %, preferably at least 85, 90 or 95 %, most preferably 100 %, to a polynucleotide which comprises at least 20, preferably at least 30 or 40, consecutive nucleotides of the nucleotide sequence according to position 673 to 5628 of SEQ ID NO: 16;
i) polynucleotides which have a sequence identity of at least 80 %, preferably at least 85, 90 or 95 %, most preferably 100 %, to a polynucleotide which comprises at least 8 or 10, preferably at least 12, consecutive nucleotides of the nucleotide sequence according to position 14 to 25 of SEQ ID NO: 10;
j) polynucleotides which have a sequence identity of at least 80 %, preferably at least 85, 90 or 95 %, most preferably 100 %, to a polynucleotide which comprises at least 8 or 10, preferably at least 12, 14, 16, 18 or 20, consecutive nucleotides of the nucleotide sequence according to position 44 to 65 of SEQ ID NO: 11;
k) polynucleotides which have a sequence identity of at least 80 %, preferably at least 85, 90 or 95 %, most preferably 100 %, to a polynucleotide which comprises at least 8 or 10, preferably at least 12, 14, 16, 18 or 20, consecutive nucleotides of the nucleotide sequence according to position 3 to 24 of SEQ ID NO: 12;
l) polynucleotides which have a sequence identity of at least 80 %, preferably at least 85, 90 or 95 %, most preferably 100 %, to a polynucleotide which comprises at least 8 or 10, preferably at least 12, 14, 16, 18 or 20, consecutive nucleotides of the nucleotide sequence according to position 45 to 66 of SEQ ID NO: 13,
m) polynucleotides (DNAs and RNAs) which are complementary to the sequences according to (a) to (I);
n) polynucleotides (DNAs and RNAs) which are complementary to the sequences according to (m);
o) polynucleotides which comprise the polynucleotides according to (a) to (n).

In a preferred embodiment of the invention combinations of said polynucleotides/markers, preferably combinations of at least 2, 3, 4, 5, 6 or 7 of said markers are used for detecting necrotic enteritis.

Another subject of the current invention is a method of detecting infection associated, in particular necrotic enteritis associated, Clostridium perfringens, the method comprising isolating microvesicles from an avian sample, and subsequently determining the presence and/or level of at least one marker indicative for the presence of infection associated, in particular necrotic enteritis associated, Clostridium perfringens in these microvesicles, wherein the presence and/or an increased level of this at least one marker in comparison to a non-infected control is indicative for the disease, in particular necrotic enteritis. In this method the at least one marker is preferably selected from the polynucleotides of List I, (a) to (h), as well as from polynucleotides (DNAs and RNAs) complementary to those sequences. More preferably the marker is selected from the polynucleotides of List II, (a) to (h), as well as from polynucleotides (DNAs and RNAs) complementary to those sequences. Even more preferably the marker is selected from the polynucleotides of List III, (a) to (h), as well as from polynucleotides (DNAs and RNAs) complementary to those sequences.

Another subject of the current invention is a method of detecting infection associated, in particular necrotic enteritis associated, avian miRNA, the method comprising isolating microvesicles from an avian sample, and subsequently determining the presence and/or level of infection associated, in particular necrotic enteritis associated, miRNA in these microvesicles, wherein the presence and/or an increased level of this at least one marker in comparison to a non-infected control is indicative for the disease, in particular necrotic enteritis. In this method the at least one marker is preferably selected from the polynucleotides of List I, (i) to (I), as well as from polynucleotides (DNAs and RNAs) complementary to those sequences. More preferably the marker is selected from the polynucleotides of List II, (i) to (I), as well as from polynucleotides (DNAs and RNAs) complementary to those sequences. Even more preferably the marker is selected from the polynucleotides of List III, (i) to (I), as well as from polynucleotides (DNAs and RNAs) complementary to those sequences.

As it is surprising that the SAM region (SEQ ID NO: 1), the swim zinc finger region (SEQ ID NO: 3), the scRNA (SEQ ID NO: 4), the CPE0956 region (SEQ ID NO: 7), the virX region (SEQ ID NO: 9) as well as mir-16 (SEQ ID NO: 10), mir-24 (SEQ ID NO: 11), mir-155 (SEQ ID NO: 12), and mir-206 (SEQ ID NO: 13) can be used as markers for determining avian necrotic enteritis, at all, a further subject of the current invention is a method of detecting avian necrotic enteritis, in particular sub-clinical avian necrotic enteritis, wherein detection of necrotic enteritis is carried out by detecting the presence and/or amount of at least one of the following sequences ("List IV") in an avian sample, preferably in microvesicles of an avian sample, in particular in comparison to a control sample of a non-infected avian:
a) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 1,
b) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 3,
c) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 4;
d) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 7;
e) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 9;
f) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40, 60 or 80, consecutive nucleotides of the nucleotide sequence as depicted in SEQ ID NO: 10;
g) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 11;
h) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 12;
i) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 13;
j) polynucleotides (DNAs and RNAs) which are complementary to the sequences according to (a) to (i);
k) polynucleotides (DNAs and RNAs) which are complementary to the sequences according to (j);
l) polynucleotides which comprise the polynucleotides according to (a) to (k).

In this method the sequences are preferably selected from the following group ("List V"):
a) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200 consecutive nucleotides of the nucleotide sequence according to position 1 to 630, in particular 420 to 630, preferably 460 to 560, of SEQ ID NO: 1,
b) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence according to position 600 to 1000, in particular 670 to 760, of SEQ ID NO: 3,
c) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence according to position 60 to 240, in particular 80 to 220, of SEQ ID NO: 4;
d) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence according to position 1 to 591, in particular 100 to 280 of SEQ ID NO: 7;
e) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence according to position 12 to 418, in particular 240 to 400 of SEQ ID NO: 9;
f) polynucleotides which have a sequence identity of at least 80 %, preferably at least 85, 90 or 95 %, most preferably 100 %, to a polynucleotide which comprises at least 8 or 10, preferably at least 12, consecutive nucleotides of the nucleotide sequence according to position 14 to 25 of SEQ ID NO: 10;
g) polynucleotides which have a sequence identity of at least 80 %, preferably at least 85, 90 or 95 %, most preferably 100 %, to a polynucleotide which which comprises at least 8 or 10, preferably at least 12, 14, 16, 18 or 20, consecutive nucleotides of the nucleotide sequence according to position 44 to 65 of SEQ ID NO: 11;
h) polynucleotides which have a sequence identity of at least 80 %, preferably at least 85, 90 or 95 %, most preferably 100 %, to a polynucleotide which comprises at least 8 or 10, preferably at least 12, 14, 16, 18 or 20, consecutive nucleotides of the nucleotide sequence according to position 3 to 24 of SEQ ID NO: 12;
i) polynucleotides which have a sequence identity of at least 80 %, preferably at least 85, 90 or 95 %, most preferably 100 %, to a polynucleotide which comprises at least 8 or 10, preferably at least 12, 14, 16, 18 or 20, consecutive nucleotides of the nucleotide sequence according to position 45 to 66 of SEQ ID NO: 13,
j) polynucleotides (DNAs and RNAs) which are complementary to the sequences according to (a) to (i);
k) polynucleotides (DNAs and RNAs) which are complementary to the sequences according to (j);
l) polynucleotides which comprise the polynucleotides according to (a) to (k).

More preferably the sequences are selected from the following group ("List VI"):
a) polynucleotides which have a sequence identity of at least 80 %, preferably at least 85, 90 or 95 %, most preferably 100 %, to a polynucleotide which comprises at least 20, preferably at least 30 or 40, consecutive nucleotides of the nucleotide sequence according to position 460 to 560 of SEQ ID NO: 1,
b) polynucleotides which have a sequence identity of at least 80 %, preferably at least 85, 90 or 95 %, most preferably 100 %, to a polynucleotide which comprises at least 20, preferably at least 30 or 40, consecutive nucleotides of the nucleotide sequence according to position 670 to 760 of SEQ ID NO: 3,
c) polynucleotides which have a sequence identity of at least 80 %, preferably at least 85, 90 or 95 %, most preferably 100 %, to a polynucleotide which comprises at least 20, preferably at least 30 or 40, consecutive nucleotides of the nucleotide sequence according to position 80 to 220 of SEQ ID NO: 4;
d) polynucleotides which have a sequence identity of at least 80 %, preferably at least 85, 90 or 95 %, most preferably 100 %, to a polynucleotide which comprises at least 20, preferably at least 30 or 40, consecutive nucleotides of the nucleotide sequence according to position 100 to 280 of SEQ ID NO: 7;
e) polynucleotides which have a sequence identity of at least 80 %, preferably at least 85, 90 or 95 %, most preferably 100 %, to a polynucleotide which comprises at least 20, preferably at least 30 or 40, consecutive nucleotides of the nucleotide sequence according to position 240 to 400 of SEQ ID NO: 9;
f) polynucleotides which have a sequence identity of at least 80 %, preferably at least 85, 90 or 95 %, most preferably 100 %, to a polynucleotide which comprises at least 8 or 10, preferably at least 12, consecutive nucleotides of the nucleotide sequence according to position 14 to 25 of SEQ ID NO: 10;
g) polynucleotides which have a sequence identity of at least 80 %, preferably at least 85, 90 or 95 %, most preferably 100 %, to a polynucleotide which which comprises at least 8 or 10, preferably at least 12, 14, 16, 18 or 20, consecutive nucleotides of the nucleotide sequence according to position 44 to 65 of SEQ ID NO: 11;
h) polynucleotides which have a sequence identity of at least 80 %, preferably at least 85, 90 or 95 %, most preferably 100 %, to a polynucleotide which comprises at least 8 or 10, preferably at least 12, 14, 16, 18 or 20, consecutive nucleotides of the nucleotide sequence according to position 3 to 24 of SEQ ID NO: 12;
i) polynucleotides which have a sequence identity of at least 80 %, preferably at least 85, 90 or 95 %, most preferably 100 %, to a polynucleotide which comprises at least 8 or 10, preferably at least 12, 14, 16, 18 or 20, consecutive nucleotides of the nucleotide sequence according to position 45 to 66 of SEQ ID NO: 13,
j) polynucleotides (DNAs and RNAs) which are complementary to the sequences according to (a) to (i);
k) polynucleotides (DNAs and RNAs) which are complementary to the sequences according to (j);
l) polynucleotides which comprise the polynucleotides according to (a) to (k).

In a preferred embodiment of the invention combinations of said polynucleotides, preferably combinations of at least 2, 3, 4, 5, 6 or 7 of said polynucleotides are used for detecting necrotic enteritis.

The avian sample according to the invention can be a tissue sample, preferably an intestinal sample, in particular a duodenal or a jejunal sample.

Preferably the avian sample is selected from avian bodily fluids and avian excrements and solutions and suspensions thereof.

The avian bodily fluid according to the invention is preferably blood, blood serum or blood plasma.

The avian excrement according to the invention is preferably selected from fecal and cecal excrements.

Suitable sample volumes are, for example, 0.1 to 20 ml, in particular 0.2 to 10 ml, preferably 0.5 to 5 ml. Suitable sample masses are, for example 0.1 to 20 g, in particular 0.2 to 10 g, preferably 0.5 to 5 g.

The avian subject according to the invention is preferably poultry.

Preferred poultry according to the invention are chickens, turkeys, ducks and geese. The poultry can be optimized for producing young stock. This type of poultry is also referred to as parent animals. Preferred parent animals are, accordingly, parent broilers, parent ducks, parent turkeys and parent geese.

The poultry according to the invention can also be selected from fancy poultry and wild fowl.

Preferred fancy poultry or wild fowl are peacocks, pheasants, partridges, guinea fowl, quails, capercaillies, grouse, pigeons and swans.

Further preferred poultry according to the invention are ostriches and parrots.

Most preferred poultry according to the invention are chickens.

As used herein, the term "polynucleotides" or "nucleic acids" refer to DNA and RNA. The polynucleotides and nucleic acids can be single stranded or double stranded.

The isolation of microvesicles from avian excrements and its subsequent use to determine an avian infection, in particular avian bacterial infection, more particular necrotic enteritis, has not been disclosed before.

Thus, a further subject of the current invention is a method of detecting an avian infection, in particular a sub-clinical avian infection, preferably an avian bacterial infection, in particular a subclinical avian bacterial infection, more preferably necrotic enteritis, in particular sub-clinical necrotic enteritis, the method comprising determining the presence and/or amount of at least one marker indicative for the disease in microvesicles isolated from avian excrements, in particular from fecal or cecal excrements, wherein the presence and/or an increased level of this at least one marker in comparison to a non-infected control is indicative for the disease. In this method the at least one marker is preferably selected from any of the polynucleotides as disclosed in List I, preferably as disclosed in List II, more preferably as disclosed in List III.

It turned out that the occurrence of markers indicative for necrotic enteritis is sample-specific.

So it was found that in fecal excrements the best marker is the SAM region, followed by scRNA, the netB region, virX region, colA region, swim zinc finger region and the CPE0956 region.

To the contrary, in cecal excrements the best marker is also the SAM region, but is followed by the swim zinc finger region, scRNA, mir206, the virX region, the netB region, the CPE0956 region and the colA region.

Further in blood samples the best marker is mir16, followed by mir155 and mir24. Thus a preferred embodiment of the current application is a method of detecting avian necrotic enteritis, in particular sub-clinical avian necrotic enteritis, the method comprising determining the level of at least one marker indicative for necrotic enteritis in avian fecal excrements, preferably in microvesicles isolated from avian fecal excrements, wherein an increased level of this at least one marker in comparison to a non-infected control is indicative for necrotic enteritis and wherein the marker is preferably selected from
a) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 1,
b) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 3,
c) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence as depicted in SEQ ID NO: 4;
d) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 5;
e) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 7;
f) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 8;
g) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 9;
h) polynucleotides (DNAs and RNAs) which are complementary to the sequences according to (a) to (g);
i) polynucleotides (DNAs and RNAs) which are complementary to the sequences according to (h);
j) polynucleotides which comprise the polynucleotides according to (a) to (i).

In this method the sequences are preferably selected from the following group:
a) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence according to position 1 to 630, in particular 420 to 630, preferably 460 to 560, of SEQ ID NO: 1,
b) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence according to position 600 to 1000, in particular 670 to 760, of SEQ ID NO: 3,
c) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence according to position 60 to 240, in particular 80 to 220, of SEQ ID NO: 4;
d) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides according to position 1300 to 1800, in particular 1400 to 1700, of (the sequence as depicted in) SEQ ID NO: 5;
e) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence according to position 1 to 591, in particular 100 to 280 of SEQ ID NO: 7;
f) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence according to position 240 to 540, in particular 300 to 540 of SEQ ID NO: 8;
g) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence according to position 12 to 418, in particular 240 to 400 of SEQ ID NO: 9;
h) polynucleotides (DNAs and RNAs) which are complementary to the sequences according to (a) to (g);
i) polynucleotides (DNAs and RNAs) which are complementary to the sequences according to (h);
j) polynucleotides which comprise the polynucleotides according to (a) to (i).

Thus another preferred embodiment of the current application is a method of detecting avian necrotic enteritis, in particular sub-clinical avian necrotic enteritis, the method comprising determining the level of at least one marker indicative for necrotic enteritis in avian cecal excrements, in particular in microvesicles isolated from avian cecal excrements, wherein an increased level of this at least one marker in comparison to a non-infected control is indicative for necrotic enteritis and wherein the marker is preferably selected from
a) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence as depicted in SEQ ID NO: 1,
b) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence as depicted in SEQ ID NO: 3,
c) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence as depicted in SEQ ID NO: 4;
d) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence as depicted in SEQ ID NO: 5;
e) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence as depicted in SEQ ID NO: 7;
f) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence as depicted in SEQ ID NO: 8;
g) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence as depicted in SEQ ID NO: 9;
h) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 13;
i) polynucleotides (DNAs and RNAs) which are complementary to the sequences according to (a) to (h);
j) polynucleotides (DNAs and RNAs) which are complementary to the sequences according to (i);
k) polynucleotides which comprise the polynucleotides according to (a) to (j).

In this method the sequences are preferably selected from the following group:
a) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence according to position 1 to 630, in particular 420 to 630, preferably 460 to 560, of SEQ ID NO: 1,
b) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence according to position 600 to 1000, in particular 670 to 760, of SEQ ID NO: 3,
c) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence according to position 60 to 240, in particular 80 to 220, of SEQ ID NO: 4;
d) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides according to position 1300 to 1800, in particular 1400 to 1700, of (the sequence as depicted in) SEQ ID NO: 5;
e) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence according to position 1 to 591, in particular 100 to 280 of SEQ ID NO: 7;
f) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence according to position 240 to 540, in particular 300 to 540 of SEQ ID NO: 8;
g) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence according to position 12 to 418, in particular 240 to 400 of SEQ ID NO: 9;
h) polynucleotides which have a sequence identity of at least 80 %, preferably at least 85, 90 or 95 %, most preferably 100 %, to a polynucleotide which comprises at least 8 or 10, preferably at least 12, 14, 16, 18 or 20, consecutive nucleotides of the nucleotide sequence according to position 45 to 66 of SEQ ID NO: 13,
i) polynucleotides (DNAs and RNAs) which are complementary to the sequences according to (a) to (h);
j) polynucleotides (DNAs and RNAs) which are complementary to the sequences according to (i);
k) polynucleotides which comprise the polynucleotides according to (a) to (j).

Thus another preferred embodiment of the current application is a method of detecting avian necrotic enteritis, in particular sub-clinical necrotic enteritis, the method comprising determining the level of at least one marker indicative for necrotic enteritis in avian blood, in particular in microvesicles isolated from avian blood, wherein an increased level of this at least one marker in comparison to a non-infected control is indicative for necrotic enteritis and wherein the at least one marker is preferably selected from
a) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40, 60 or 80, consecutive nucleotides of the nucleotide sequence as depicted in SEQ ID NO: 10;
b) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 11;
c) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 12;
d) polynucleotides (DNAs and RNAs) which are complementary to the sequences according to (a) to (c);
e) polynucleotides (DNAs and RNAs) which are complementary to the sequences according to (d);
f) polynucleotides which comprise the polynucleotides according to (a) to (e).

In this method the sequences are preferably selected from the following group:
a) polynucleotides which have a sequence identity of at least 80 %, preferably at least 85, 90 or 95 %, most preferably 100 %, to a polynucleotide which comprises at least 8 or 10, preferably at least 12, consecutive nucleotides of the nucleotide sequence according to position 14 to 25 of SEQ ID NO: 10;
b) polynucleotides which have a sequence identity of at least 80 %, preferably at least 85, 90 or 95 %, most preferably 100 %, to a polynucleotide which which comprises at least 8 or 10, preferably at least 12, 14, 16, 18 or 20, consecutive nucleotides of the nucleotide sequence according to position 44 to 65 of SEQ ID NO: 11;
c) polynucleotides which have a sequence identity of at least 80 %, preferably at least 85, 90 or 95 %, most preferably 100 %, to a polynucleotide which comprises at least 8 or 10, preferably at least 12, 14, 16, 18 or 20, consecutive nucleotides of the nucleotide sequence according to position 3 to 24 of SEQ ID NO: 12;
d) polynucleotides (DNAs and RNAs) which are complementary to the sequences according to (a) to (c);
e) polynucleotides (DNAs and RNAs) which are complementary to the sequences according to (d);
f) polynucleotides which comprise the polynucleotides according to (a) to (e).

A further subject of the current invention is a method of treatment of necrotic enteritis comprising performing the method of detecting necrotic enteritis as described before and subsequently administering a therapeutic agent for the treatment of necrotic enteritis.

A further subject of the current invention is also a nucleotide array for detecting avian necrotic enteritis, wherein the nucleotide array comprises at least one marker, preferably at least 2, 3, 4 or 5 markers, selected from the polynucleotides as depicted in List IV, preferably as depicted in List V, more preferably as depicted in List VI.

A further subject of the current invention is also a kit comprising a set of oligonucleotides for amplifying at least one polynucleotide associated with necrotic enteritis, wherein the at least one polynucleotide is selected from the polynucleotides as depicted in List IV, preferably as depicted in List V, more preferably as depicted in List VI.

NetB and TpeL have already been discussed to be major toxins involved in the pathogenesis of necrotic enteritis (Keyburn et al, 2010; WO 2008/148166; Shojadoost et a1.,2012)

ColA stands for Collagenese A of Clostridium perfringens; this gene might be associated with the pathogenesis of NE because of its similarity to the so called kappa-toxin, one of the supposed major toxins of this bacterium (Obana et al., 2013).

Nevertheless, the occurrence as well as the deregulation of these genes in microvesicles could not have been expected.

The virX gene is known to regulate the plx, colA, and pfoA genes in C. perfringens (Ohtani et al., 2002), but its involvement in the pathogenesis of NE has not been reported before.

The small cytoplasmic RNA (scRNA), a member of an evolutionarily conserved signal-recognition-particle-like RNA family involved in the sporulation mechanism of Clostridium perfringens (Nakamura et al., 1995) has also not been reported previously to be in direct association with NE in chickens.

Furthermore, the involvement of the SAM domain, the swim zinc finger domain and the CPE0956 region in the pathogenesis of necrotic enteritis has not been disclosed before and could also not have been expected as these sequences are sequences with previously unknown functions. A further subject of the current inventions are therefore polynucleotides, preferably encoding a polypeptide with toxin activity and/or a virulence factor involved in necrotic enteritis, selected from the group consisting of
a) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 1;
b) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 3;
c) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 7;
d) polynucleotides (DNAs and RNAs) which are complementary to the sequences according to (a) to (c);
e) polynucleotides which comprise the polynucleotides according to (a) to (d).

Particularly preferred are polynucleotides selected from the group consisting of
a) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200 consecutive nucleotides of the nucleotide sequence according to position 1 to 630, in particular 420 to 630, preferably 460 to 560, of SEQ ID NO: 1;
b) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence according to position 600 to 1000, in particular 670 to 760, of SEQ ID NO: 3;
c) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence according to position 1 to 591, in particular 100 to 280 of SEQ ID NO: 7;
d) polynucleotides (DNAs and RNAs) which are complementary to the sequences according to (a) to (c);
e) polynucleotides which comprise the polynucleotides according to (a) to (d).

A further subject of the current invention are therefore also polypeptides, which are preferably toxins and/or virulence factors involved in necrotic enteritis, selected from the group consisting of
a) polypeptides which have a sequence identity of at least 80 %, preferably at least 85, 90 or 95 %, most preferably 100 %, to a polypeptide which comprises at least 10, preferably at least 15, 20, 25, 40, 60, 100, 150, 180 or 200, in particular all, consecutive amino acids of the amino acid sequence as depicted in SEQ ID NO: 2;
b) polypeptides which have a sequence identity of at least 80 %, preferably at least 85, 90 or 95 %, most preferably 100 %, to a polypeptide which comprises at least 10, preferably at least 15, 20, 25, 40, 60 or 70, in particular all, consecutive amino acids of the amino acid sequence as depicted in SEQ ID NO: 14;
c) polypeptides which have a sequence identity of at least 80 %, preferably at least 85, 90 or 95 %, most preferably 100 %, to a polypeptide which comprises at least 10, preferably at least 15, 20, 25, 40, 60, 100, 150, 180 or 190, in particular all, consecutive amino acids of the amino acid sequence as depicted in SEQ ID NO: 15;
d) polypeptides which comprise the polypeptides according to (a) to (c).

A further subject of the current invention is therefore also a method of raising an immune response in an animal; the method comprising administering to the animal a polypeptide selected from the group consisting of
a) polypeptides which have a sequence identity of at least 80 %, preferably at least 85, 90 or 95 %, most preferably 100 %, to a polypeptide which comprises at least 10, preferably at least 15, 20, 25, 40, 60, 100, 150, 180 or 200, in particular all, consecutive amino acids of the amino acid sequence as depicted in SEQ ID NO: 2;
b) polypeptides which have a sequence identity of at least 80 %, preferably at least 85, 90 or 95 %, most preferably 100 %, to a polypeptide which comprises at least 10, preferably at least 15, 20, 25, 40, 60 or 70, in particular all, consecutive amino acids of the amino acid sequence as depicted in SEQ ID NO: 14;
c) polypeptides which have a sequence identity of at least 80 %, preferably at least 85, 90 or 95 %, most preferably 100 %, to a polypeptide which comprises at least 10, preferably at least 15, 20, 25, 40, 60, 100, 150, 180 or 190, in particular all, consecutive amino acids of the amino acid sequence as depicted in SEQ ID NO: 15;
d) polypeptides which comprise the polypeptides according to (a) to (c).

The polypeptides according to the invention preferably exhibit toxin activity and are preferably obtainable from Clostridium perfringens. More preferably the polypeptides according to the invention are NE associated virulence factors.

A further subject of the current invention are vectors, in particular cloning and expression vectors as well as viral and plasmid vectors, comprising a polynucleotide according to the invention.

A further subject of the current invention are also cells, in particular bacterial cells, preferably E. coli cells, comprising a polynucleotide, a polypeptide and/or a vector according to the invention.

A further subject of the current invention is a polyclonal or monoclonal antibody or a fragment thereof, which binds specifically to a polypeptide with toxin activity according to the invention, in particular to NE associated virulence factors according to the invention.

A further subject of the current invention is a composition, comprising a polynucleotide, a polypeptide, a vector, a cell or an antibody according to the invention.

A further subject of the current invention is a diagnostic kit comprising a polynucleotide or an antibody according to the invention. The kit may further comprise a capture surface apparatus suitable to separate microvesicles from a biological sample from unwanted particles, debris, and small molecules, as explained in more detail below. The kit might also include instructions for its use, in particular for using the components in the optional isolation and lysis of microvesicles and the subsequent qualitative or quantitative determination of the presence of at least one of the markers according to the invention.

A further subject of the current invention is an immunogenic composition comprising a polypeptide according to the invention and an adjuvant and/or a pharmaceutically acceptable carrier.

A further subject of the current invention is also a vaccine, comprising a polypeptide according to the invention and preferably an adjuvant and/or a pharmaceutically acceptable carrier.

Microvesicles according to the invention comprise membrane vesicles from bacteria, in particular from Clostridium perfringens, as well as from the host, in particular chicken.

The eukaryotic version of membrane vesicles is also called exosomes. Exosomes have typically a diameter of 30-200 nm. They are shed into all biofluids and carry many intact proteins and oligonucleotides, as they are protected from degredation by RNAses (Koga et al., 2011)

Exosomes have been studied in particular in cancer and immune cells.

Microvesicles from bacteria are also called extracellular membrane vesicles (MVs) or outer membrane vesicles (OMVs). They are spherical bilayer structures with average diameter of typically 20-500 nm. Bacterial microvesicles have first been described for Gram-negative bacteria, but later also for some Gram-positive bacteria. In a recent study, it was reported that microvesicles are produced and released also by C. perfringens type A strains, triggering innate and adaptive immune responses. It was further found out in the course of this study that important virulence factors like alpha-toxin and NetB were not present in the membrane vesicles, whereas beta2-toxin was found in the membrane vesicles (Jiang et al., (2014).

MicroRNAs (miRNAs) are small, non-coding RNAs that are usually 18-23 nucleotides in size and have been suggested to play a critical role in the regulation of gene expression. In particular the genes associated with immune responses have been reported to be highly targeted by miRNAs. In chickens miRNA expression has been studied in embryo developmental processes, germ cell development, immune organs and diseases.

In recent studies the miRNA profile of two different highly inbred White Leghorn chicken lines infected by C. perfringens was investigated (Dinh et al., 2014; Hong et al., 2014). RNA analysis was carried out with tissue from spleen and intestinal mucosa. The results suggest that some miRNAs are differentially altered in response to necrotic enteritis and that they modulate the expression of their target genes. Microvesicles were not investigated in these studies.

The miRNAs as found to be correlative to necrotic enteritis according to the current invention were investigated before by Wang et al. in connection with avian influenza virus infected broilers (Wang et al., 2012). Wang et al. found that the miRNAs miR-155, miR-16-1 and miR-24 are expressed stronger in the lungs of infected broilers than in those of non-infected ones, but they found further that the miR-206 is, to the contrary, stronger expressed in non-infected ones than in infected ones. Thus the use of miR-206 as marker for a bacterial infection is disclosed in the current application for the first time. Further in contrast to Wang et al. according to the current invention all miRNAs were detected in microvesicles, not in tissue.

Thus a further subject of the current invention is a method of detecting an avian bacterial infection, in particular avian necrotic enteritis, the method comprising isolating microvesicles from an avian sample and subsequently determining the level of at least one avian marker indicative for the infection, wherein an increased level of this at least one avian marker in comparison to a non-infected control is indicative for the infection and wherein the at least one avian marker is an avian miRNA and is preferably selected from the group consisting of
a) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40, 60 or 80, consecutive nucleotides of the nucleotide sequence as depicted in SEQ ID NO: 10;
b) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 11;
c) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 12;
d) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 13;
e) polynucleotides (DNAs and RNAs) which are complementary to the sequences according to (a) to (d);
f) polynucleotides (DNAs and RNAs) which are complementary to the sequences according to (e);
g) polynucleotides which comprise the polynucleotides according to (a) to (f).

In this method the sequences are preferably selected from the following group:
a) polynucleotides which have a sequence identity of at least 80 %, preferably at least 85, 90 or 95 %, most preferably 100 %, to a polynucleotide which comprises at least 8 or 10, preferably at least 12, consecutive nucleotides of the nucleotide sequence according to position 14 to 25 of SEQ ID NO: 10;
b) polynucleotides which have a sequence identity of at least 80 %, preferably at least 85, 90 or 95 %, most preferably 100 %, to a polynucleotide which which comprises at least 8 or 10, preferably at least 12, 14, 16, 18 or 20, consecutive nucleotides of the nucleotide sequence according to position 44 to 65 of SEQ ID NO: 11;
c) polynucleotides which have a sequence identity of at least 80 %, preferably at least 85, 90 or 95 %, most preferably 100 %, to a polynucleotide which comprises at least 8 or 10, preferably at least 12, 14, 16, 18 or 20, consecutive nucleotides of the nucleotide sequence according to position 3 to 24 of SEQ ID NO: 12;
d) polynucleotides which have a sequence identity of at least 80 %, preferably at least 85, 90 or 95 %, most preferably 100 %, to a polynucleotide which comprises at least 8 or 10, preferably at least 12, 14, 16, 18 or 20, consecutive nucleotides of the nucleotide sequence according to position 45 to 66 of SEQ ID NO: 13,
e) polynucleotides (DNAs and RNAs) which are complementary to the sequences according to (a) to (d);
f) polynucleotides (DNAs and RNAs) which are complementary to the sequences according to (e);
g) polynucleotides which comprise the polynucleotides according to (a) to (f).

As mir-206 could be for the first time be identified as marker for avian infections, at all, a further subject of the current invention is a method for detecting an avian infection, preferably an avian bacterial infection, more preferably avian necrotic enteritis, wherein an increased level of at least one avian marker in comparison to a non-infected control is indicative for the infection and wherein the at least one avian marker is selected from the group consisting of
a) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 13;
b) polynucleotides (DNAs and RNAs) which are complementary to the sequences according to (a);
c) polynucleotides which comprise the polynucleotides according to (a) or (b).

In this method the sequences are preferably selected from the following group:
a) polynucleotides which have a sequence identity of at least 80 %, preferably at least 85, 90 or 95 %, most preferably 100 %, to a polynucleotide which comprises at least 8 or 10, preferably at least 12, 14, 16, 18 or 20, consecutive nucleotides of the nucleotide sequence according to position 45 to 66 of SEQ ID NO: 13,
b) polynucleotides (DNAs and RNAs) which are complementary to the sequences according to (a);
c) polynucleotides which comprise the polynucleotides according to (a) or (b).

Applications of the methods according to the invention are for example (i) aiding in the diagnosis and/or prognosis of avian necrotic enteritis, (ii) monitoring the progress or reoccurrence of avian necrotic enteritis, or (iii) aiding in the evaluation of treatment efficacy for an avian subject undergoing or contemplating treatment for necrotic enteritis; wherein the presence or absence of one or more of the biomarkers mentioned before in the nucleic acid extraction obtained from the method is determined, and the one or more biomarkers are associated with the diagnosis, progress or reoccurrence, or treatment efficacy, respectively, of necrotic enteritis.

Applications of the invention in particular help to avoid loss in avian performance like weight gain and feed conversion.

### Isolation of microvesicles

The isolation of microvesicles from a biological sample prior to extraction of nucleic acids is advantageous, because nucleic acid-containing microvesicles produce significantly higher yields of nucleic acid species with higher integrity as compared to the yield/integrity obtained by extracting nucleic acids directly from the fluid sample or excrement without first isolating microvesicles. Further it is also possible to detect nucleic acids which are expressed at low levels. The isolation of microvesicles also allows the exclusion of proteins, lipids, cell debris, cells and other potential contaminants and PCR inhibitors that are naturally found within biological samples.

Isolation of membrane vesicles from biological samples and subsequent extraction of the nucleic acids can be done for example by ultracentrifugation, e.g., spinning at more than 10,000 g for 1-3 hours, followed by removal of the supernatant, washing the pellet, lysing the pellet and purifying the nucleic acids on a column. Alternative methods are ultrafiltration, e.g., using 100 kD filters, polymer precipitation techniques, and/or filtration based on size.

Further alternative methods are differential centrifugation as described by Raposo et al (1996), Skog et. al( 2008) and Nilsson et. al ( 2009); ion exchange and/or gel permeation chromatography as described in US Patent Nos. 6,899,863 and 6,812,023; sucrose density gradients or organelle electrophoresis as described in U.S. Patent No. 7,198,923; magnetic activated cell sorting (MACS) as described by Taylor and Gercel-Taylor( 2008); nanomembrane ultrafiltration concentration as described by Cheruvanky et al(2007); Percoll gradient isolation as described by Miranda et al ( 2010); and, isolation by a microfluidic device Chen et al(2010).

Capture surface based isolation of microvesicles according to WO 2014/107571

In a preferred embodiment the microvesicle fraction is bound to a capture surface, in particular to a membrane, a filter or to a surface modified bead as disclosed in WO 2014/107571.

The biological sample is preferably dissolved in a loading buffer, which has preferably a pH of 4-8, more preferably 5-7. Thus, binding to the capture surface takes preferably place at a pH of 4-8, more preferably 5-7. Preferably, after contacting the biological sample with the capture surface, one or more wash steps are performed.

The loading and wash buffers which can be employed according to the invention can be of high or low ionic strength. Thus, the salt concentration, e.g., NaCI concentration, can be from 0 to 2.4M. Preferably, the buffers include one or more of the following components: Tris, Bis-Tris, Bis-Tris-Propane, Imidazole, Citrate, Methyl Malonic Acid, Acetic Acid, Ethanolamine, Diethanolamine, Triethanolamine (TEA) and Sodium phosphate.

Washing preferably takes place by using wash buffer comprising 250 mM Bis Tris Propane and exhibiting a pH of 6.5-7.0.

By adding detergents to the wash buffer nonspecific binding is suppressed. Detergents suitable for use include, but are not limited to, sodium dodecyl sulfate (SDS), Tween-20, Tween-80, Triton X-100, Nonidet P-40 (NP-40), Brij-35, Brij-58, octyl glucoside, octyl thioglucoside, CHAPS or CHAPSO.

After washing of the capture surface the nucleic acid can be released by lysing the fixed microvesicles. For lysing the fixed microvesicles preferably a phenol-based lysis buffer is used, like QIAzol^{®} lysis reagent (Qiagen, Germany). Purification of the nucleic acid can then be done by chloroform extraction using PLG tubes, followed by ethanol conditioning. The nucleic acid can then be bound to a silica column before washing and eluting.

The lysis of microvesicles and extraction of nucleic acids may also be achieved by using a commercially available Qiagen R easy Plus kit, a commercially available Qiagen miR easy kit or by using phenolchloroform according to standard procedures and techniques known in the art. Such methods may also utilize a nucleic acid-binding column to capture the nucleic acids contained within the microvesicles. Once bound, the nucleic acids can then be eluted using a buffer or solution suitable to disrupt the interaction between the nucleic acids and the binding column. The nucleic acid extraction methods may also include the use of further agents like an RNase inhibitor such as Superase-In (commercially available from Ambion Inc.) or RNaseINplus (commercially available from Promega Corp.); a protease (which may also function as an RNase inhibitor); DNase; a reducing agent; a decoy substrate such as a synthetic RNA and/or carrier RNA; a soluble receptor that can bind RNase; a small interfering RNA (siRNA); an RNA binding molecule, such as an anti-RNA antibody, a basic protein or a chaperone protein; an RNase denaturing substance, such as a high osmolarity solution, a detergent, or a combination thereof. The RNAse inhibitors as well as the further agents may be added to the biological sample, and/or to the isolated microvesicle fraction, prior to extracting nucleic acids.

These further agents may exert their functions in various ways, e.g., through inhibiting RNase activity (e.g., RNase inhibitors), through a ubiquitous degradation of proteins (e.g., proteases), or through a chaperone protein (e.g., a RNA-binding protein) that binds and protects RNAs. In all instances, such extraction enhancement agents remove or at least mitigate some or all of the adverse factors in the biological sample or associated with the isolated particles that would otherwise prevent or interfere with the high quality extraction of nucleic acids from the isolated particles.

The quantification of 18S and 28S rRNAs extracted can be used to determine the quality of the nucleic acid extraction.

The capture surface can be neutral, negatively charged or a positively charged. That means, in principle any kind of capture surface can be used. Depending on the membrane material, the pore sizes of the membrane can range from 20 nm to 5 µm.

In particular the capture surface can be a commercially available membrane like Mustang® lon Exchange Membrane from PALL Corporation; Vivapure® Q, Vivapure® Q Maxi H; Sartobind® D, Sartobind (S), Sartobind® Q, Sartobind® IDA or Sartobind^{®} Aldehyde from Sartorius AG; Whatman® DE81 from Sigma; Fast Trap Virus Purification column from EMD Millipore; or Thermo Scientific* Pierce Strong Cation and Anion Exchange Spin Columns.

When a membrane is used for fixing the nucleic acids, then the membrane can be made from a variety of suitable materials, for example it can be a polyethersulfone (PES) (e.g., from Millipore or PALL Corp.) or regenerated cellulose (RC) (e.g., from Sartorius or Pierce).

When a negatively charged membrane is used, then the capture surface is preferably an S membrane, which is a negatively charged membrane and is a cation exchanger with sulfonic acid groups. Preferably, the S membrane is functionalized with sulfonic acid, R-CH₂-SO₃". Alternatively, the negatively charged capture surface is a D membrane, which is a weak basic anion exchanger with diethylamine groups, R-CH₂-NFT(C₂H₅)₂. Alternatively, the capture surface is a metal chelate membrane. For example, the membrane is an IDA membrane, functionalized with minodiacetic acid -N(CH₂COOH")₂. In some embodiments, the capture surface is a microporous membrane, functionalized with aldehyde groups, -CHO. In other embodiments, the membrane is a weak basic anion exchanger, with diethylamino ethyl (DEAE) cellulose.

When a neutral capture surface is used, then the capture surface is preferably a filter selected from the group consisting of 20nm neutral PES syringe filtration (Tisch and Exomir), 30nm neutral PES syringe filtration (Sterlitech), 50nm neutral PES syringe filtration (Sterlitech), 0.2um neutral PES homemade spin column filtration (Pall), 0.8um neutral PES homemade spin column filtration (Pall) and 0.8um neutral PES syringe filtration (Pall). In embodiments where the capture surface is neutral, preferably the neutral capture surface is not housed in a syringe filter. The charged surface is preferably selected from the group consisting of 0.65um positively charged Q PES vacuum filtration, 3-5um positively charged Q RC spin column filtration, 0.8um positively charged Q PES homemade spin column filtration, 0.8um positively charged Q PES syringe filtration, 0.8um negatively charged S PES homemade spin column filtration, 0.8um negatively charged S PES syringe filtration, and 50nm negatively charged nylon syringe filtration.

In the preferred embodiment, when the surface is a positively charged membrane, a Q membrane, which is a positively charged membrane and is an anion exchanger with quaternary amines, can be used. In a preferred embodiment the Q membrane is functionalized with quaternary ammonium, R-CH₂-N⁺(CH₃)₃.

In a very preferred embodiment the positively charged membrane is a regenerated cellulose, strong basic anion exchanger ("RC/SBAE") membrane, which is an anion exchanger with quaternary amines. In a preferred embodiment the RC/SBAE membrane is functionalized with quaternary ammonium, R-CH₂-N⁺(CH₃)₃. The pore size of the membrane is preferably at least 3 µm.

The capture surface, e.g., membrane, can be housed within a device used for centrifugation; e.g. spin columns, or for vacuum application e.g. vacuum filter holders, or for filtration with pressure e.g. syringe filters.

In a spin-column based purification process preferably more than 1 membrane, preferably 2 to 5 membranes, are used in parallel in a tube, wherein the membranes are preferably all directly adjacent to one another at one end of the column. The collection tube may be commercially available, like a 50ml Falcon tube. The column is preferably suitable for spinning, i.e., the size is compatible with standard centrifuge and micro-centrifuge machines.

Instead of a membrane also a bead with the same surface modification can be used for capturing the nucleic acid. If a bead is used, it can be magnetic or non-magnetic.

If blood is used as a sample, blood can for example be collected into K2 EDTA tubes and mixed well with complete inversions. Then the tubes are centrifuged at 1300xg for 10 min to separate the blood cells and plasma. Then plasma is removed and filtered through a 0.8µm filter to remove cell debris and platelets. All plasma samples are then aliquoted into 1ml cryovials and stored at -80°C until use.

Before RNA isolation, the membrane is preferably conditioned by passing through equilibrium buffer. The thawed plasma sample is diluted with loading buffer. The diluted plasma sample is slowly passed through the membrane that adsorbs the microvesicles. The membrane is then washed with a wash buffer to remove any weakly bound plasma components. Then a lysis reagent is passed through the membrane to lyse the microvesicles. RNA is isolated using the miRNeasy kit (Qiagen).

RNA can be assessed for quality and concentration with the 2100 Bioanalyzer (Agilent) using a RNA 6000 Pico Chip. The relative quantity of the extracted RNA is measured by RT-qPCR using selected human gene expression assays from Applied Biosystems (Taqman Assay). In preferred embodiments, in particular when solubilized fecal or cecal samples are used, a pre-processing step prior to isolation, purification or enrichment of the microvesicles is performed to remove large unwanted particles, cells and/or cell debris and other contaminants present in the biological sample. The preprocessing steps may be achieved through one or more centrifugation steps (e.g., differential centrifugation) or one or more filtration steps (e.g., ultrafiltration), or a combination thereof. Where more than one centrifugation pre-processing steps are performed, the biological sample may be centrifuged first at a lower speed and then at a higher speed. If desired, further suitable centrifugation pre-processing steps may be carried out. Alternatively or in addition to the one or more centrifugation pre-processing steps, the biological sample may be filtered. For example, a biological sample may be first centrifuged at a low speed of about 100-500g, preferably 250-300g, and after that at a higher speed of about 2,000 to about 200,000 g, preferably at about 20,000g, for 10 minutes to about 1 hour, preferably at a temperature of 0-10°C, to remove large unwanted particles; the sample can then be filtered, for example, by using a filter with an exclusion size of 0.1 to 1.0 µm.

The microvesicles in the pellet obtained by centrifugation at higher speeds may be reconstituted with a smaller volume of a suitable buffer for the subsequent steps of the process. The concentration step may also be performed by ultrafiltration. In fact, this ultrafiltration both concentrates the biological sample and performs an additional purification of the microvesicle fraction. In another embodiment, the filtration is an ultrafiltration, preferably a tangential ultrafiltration. Tangential ultrafiltration consists of concentrating and fractionating a solution between two compartments (filtrate and retentate), separated by membranes of determined cut-off thresholds. The separation is carried out by applying a flow in the retentate compartment and a transmembrane pressure between this compartment and the filtrate compartment. Different systems may be used to perform the ultrafiltration, such as spiral membranes (Millipore, Amicon), flat membranes or hollow fibers (Amicon, Millipore, Sartorius, Pall, GF, Sepracor). Within the scope of the invention, the use of membranes with a cut-off threshold below 1000 kDa, preferably between 100 kDa and 1000 kDa, or even more preferably between 100 kDa and 600 kDa, is advantageous.

As additional or alternative preprocessing steps size-exclusion chromatography, gel permeation chromatography or affinity chromatography might be carried out before or after contacting the biological sample with the capture surface.

To perform the gel permeation chromatography step, a support selected from silica, acrylamide, agarose, dextran, ethylene glycol-methacrylate copolymer or mixtures thereof, e.g., agarose-dextran mixtures, are preferably used. For example, such supports include, but are not limited to: SUPERDEX® 200HR (Pharmacia), TSK G6000 (TosoHaas) or SEPHACRYL® S (Pharmacia).

Affinity chromatography can be accomplished, for example, by using different supports, resins, beads, antibodies, aptamers, aptamer analogs, molecularly imprinted polymers, or other molecules known in the art that specifically target desired surface molecules on microvesicles.

Optionally, control particles may be added to the sample prior to microvesicle isolation or nucleic acid extraction to serve as an internal control to evaluate the efficiency or quality of microvesicle purification and/or nucleic acid extraction. These control particles include Q-beta bacteriophage, virus particles, or any other particle that contains control nucleic acids (e.g., at least one control target gene) that may be naturally occurring or engineered by recombinant DNA techniques. The control target gene can be quantified using real-time PCR analysis.

Preferably, the control particle is a Q-beta bacteriophage, referred to herein as "Q-beta particle." The Q-beta particle may be a naturally-occurring virus particle or may be a recombinant or engineered virus, in which at least one component of the virus particle (e.g., a portion of the genome or coat protein) is synthesized by recombinant DNA or molecular biology techniques known in the art. Q-beta is a member of the leviviridae family, characterized by a linear, single-stranded RNA genome that consists of 3 genes encoding four viral proteins: a coat protein, a maturation protein, a lysis protein, and RNA replicase. Due to its similar size to average microvesicles, Q-beta can be easily purified from a biological sample using the same purification methods used to isolate microvesicles, as described herein. In addition, the low complexity of the Q-beta viral single-stranded gene structure is advantageous for its use as a control in amplification-based nucleic acid assays. The Q-beta particle contains a control target gene or control target sequence to be detected or measured for the quantification of the amount of Q-beta particle in a sample. For example, the control target gene is the Q-beta coat protein gene. After addition of the Q-beta particles to the biological sample, the nucleic acids from the Q-beta particle are extracted along with the nucleic acids from the biological sample using the extraction methods and/or kits described herein. Detection of the Q-beta control target gene can be determined by RT-PCR analysis, for example, simultaneously with the biomarkers of interest (i.e., BRAF). A standard curve of at least 2, 3, or 4 known concentrations in 10-fold dilution of a control target gene can be used to determine copy number. The copy number detected and the quantity of Q-beta particle added can be compared to determine the quality of the isolation and/or extraction process.

Thus, 50, 100, 150, 200, 250, 300, 350, 400, 450, 500, 1,000 or 5,000 copies of Q-beta particles added to a bodily fluid sample. In a preferred embodiment, 100 copies of Q-beta particles are added to a bodily fluid sample. The copy number of Q-beta particles can be calculated based on the ability of the Q-beta bacteriophage to infect target cells. Thus, the copy number of Q-beta particles is correlated to the colony forming units of the Q-beta bacteriophage.

### Detection of nucleic acid biomarkers

For detection of RNA, the RNA is preferably reverse-transcribed into complementary DNA (cDNA) before further amplification. Such reverse transcription may be performed alone or in combination with an amplification step. One example of a method combining reverse transcription and amplification steps is reverse transcription polymerase chain reaction (RT-PCT), which may be further modified to be quantitative, e.g. quantitative RT-PCT as described in US Patent No. 5,639,606. Another example of the method comprises two separate steps: a first of reverse transcription to convert RNA into cDNA and a second step of quantifying the amount of cDNA using quantitative PCR.

RT-PCR analysis determines a Ct (cycle threshold) value for each reaction. In RT-PCR, a positive reaction is detected by accumulation of a fluorescence signal. The Ct value is defined as the number of cycles required for the fluorescent signal to cross the threshold (i.e., exceeds background level). Ct levels are inversely proportional to the amount of target nucleic acid, or control nucleic acid, in the sample (i.e., the lower the Ct level, the greater the amount of control nucleic acid in the sample). Alternatively, the copy number of the control nucleic acid can be measured using another art-recognized techniques.

The analysis of nucleic acids present in the isolated particles is quantitative and/or qualitative. For quantitative analysis, the amounts (expression levels), either relative or absolute, of specific nucleic acids of interest within the isolated particles are measured with methods known in the art. For qualitative analysis, the species of specific nucleic acids of interest within the isolated microvesicles are identified with methods known in the art.

### Working examples

### Inoculation preparation

Clostridium perfringens isolate being positive for netB and tpeL was removed from -80°C storage and aseptically streaked on trypticase soy agar plates containing 5% sheep blood (TSA II). Plates were incubated at 37°C overnight under anaerobic conditions. Fifteen hours prior to the start of the inoculum culture, an isolated colony from the overnight plate was inoculated into 50ml pre-reduced BHI broth and incubated at 37□C under anaerobic conditions for about 15hours. Then 50µl of the culture was added to 2 x 100 ml of pre-reduced BHI broth. The resulting broth-cultures were staggered so that the high dose and low dose reached their final concentrations at the same time (109 cfu /ml and 106cfu/ml respectively). Aliquots of each culture were taken at the end of the growth period and serially plated onto TSA II agar +5% sheep's blood to verify bacterial concentration.

### Animal experiment

Seven hundred and twenty (720) male broilers raised in 36 battery pens for 30 days. Birds were equally and randomly assigned (9 pens each) into one of four treatment groups (negative control, sham, low dose gavage and high dose gavage). The birds were fed with standard corn/soy feed without antimicrobials. On the 13th day of rearing, over or under-weight birds were removed from all pens and excluded from the experiment. The bird with normal weight range were then challenged orally with 1 ml of C. *perfringens* strain harbouring the netB plasmid and being positive for the Tpel gene succesively at days 14,15 and 16. The sham group was challenged with 1ml of PBS, whereas the negative control group was not subjected to any stress or pathogen challenge. Before the first inoculation (day 14), 3 birds from each pen were randomly selected, sacrificed for the collection of blood and digesta samples. Twelve hours after the second challenge, on day 15, 3 birds were again sacrificed from each pen for sample blood and digesta. The gut contents of all sacrificed birds were cultured overnight to determine the ratio of sporulated C. *perfringens* to the vegetative cells as previously described (Heikinheimo et al, 2004). To assess whether challenge was successful, PCR of *NetB* and *TpeL* genes of the gut contents was performed. At least 10 birds were left in each of the pens until the end of the trial. Excreta samples were collected at intervals until study was terminated on day 20 when all birds were sacrificed and three pooled blood samples were obtained from each group. GITs of birds were examined for NE typical signs/lesions and necropsies of the gastro-intestinal tract were taken from duodenum and jejunum for histopathological analysis. Broiler performance indicators like mortality rate, feed consumption and group pathology were determined (table 3).

### Sample collection

### 1. Fecal and Cecal Excreta Sample Collections

Sheets of paper were placed in the pens to collect pooled fecal and pooled cecal samples every 2 hours after the first challenge until 12 hours after the second inoculation (day 15). Subsequently, samples were collected every 4 hours until day 17. At the end of the trial on day 20, fecal and cecal samples were also collected. During any of the collection event at least 1 gram of fecal and cecal was obtained per pen, and samples were store at -80°C.

### 2. Blood Collection

Blood was collected directly from the heart using a 18 gauge needle. About 5-6 ml of whole blood was collected from 3 randomly selected birds per pen and transferred to collection tubes containing EDTA. Cells are removed from plasma by centrifugation for 10 minutes at 1,000-2,000 x g using a refrigerated centrifuge. Following centrifugation, the liquid component (plasma) was immediately transferred into a clean polypropylene tube and stored at -20°C.

### 3. Gut contents collection

Gut content was collected from a gut approximately 20 cm in length. Gut content was expelled and excised into PBS + 20% glycerol and immediately frozen at -20°C. Samples have then been used for quantitation of vegetative versus sporulated C. *perfringens* and PCR detection of netB and tpeL.

### Histology of chicken's intestine

### 1. Tissue collection and processing

A 5 cm section following the duodenal loop was removed from each bird and placed in 10% formalin, and the container was gently agitated to dislodge feed to allow formalin getting into the gut. After fixation, tissues were washed well in several changes of Phosphate Buffered Saline (PBS) and placed in 70% ethanol for storage. For the processing of tissues and infiltration of tissues with paraffin before making 5uM section, tissues were dehydrated by washing 1x for 2 min in 70% and 95% ethanol solutions successively, and then 2x for 2mins in 100% ethanol solution. Finally, tissues were rinsed 2x for 1 minute in Xylene before being embedded in paraffin. The processed tissue was then oriented in a mold, embedded in a paraffin block and cut in a microtome to generate sections of 5µM. Tissue slices are floated onto Poly-L-lysine (PLL) modified slides. The slides are dried and placed in an oven to "bake" the paraffin.

### 2. Staining of tissue sections with Harris Hematoxylin and Eosin.

In brief, sections were rehydrated by successively decreasing concentration of ethanol: 2x for 3min each in 100% ethanol, 2x for 3 mins each in 95% ethanol and 1x for 3 minutes in 70% ethanol. Slides were then rinsed for 5 minutes in distilled water and then stained for 6 minutes with Hematoxylin solution (Harris Hematoxylin, Sigma, HHS-32). Slides were rinsed in running tap water for about 20 minutes and decolorized in acid alcohol (95% Ethanol, 2578 ml dH₂O, 950ml HCL, 9ml) for 1-3 seconds. Slides were rinsed again with running tap water for 5 minutes then immersed in lithium carbonate for 3 seconds and then rinsed with tap water for 5 minutes. Tissues were counterstained with eosin working solution (100ml stock eosin (1% aqueous Eosin-Y and 1% aqueous Phloxin B), 10 ml stock Phloxin B, 780 ml 95% Ethanol, 4 ml glacial Acetic Acid) for 15 seconds and rehydrated by successive incubation in ethanol solutions (2 for 3 minutes each in 95% ethanol solution and 2x for 3minutes each in 100% ethanol solution). Finally, tissues were incubated in xylene solution for 5 minutes and then mounted with cytoseal for analysis.

### Detection of Clostridium perfingens netB and tpeL genes by PCR

**Table 1. primer pairs used for the detection of netB and tpeL genes in Clostridium perfringens isolates from chicken's intestine**

| **Primer ID** | **Sequence** | **Target Gene** | **Product Length** |
|---|---|---|---|
| netB5F | CGCTTCACATAAAGGTTGGAAGGC | *netB* | 316 |
| netB5R | TCCAGCACCAGCAG I I I TTCCT | *netB* | 316 |
| AKP80F | ATATAGAGTCAAGCAGTGGAG | *tpeL* | 466 |
| AKP81R | GGAATACCACTTGATATACCTG | *tpeL* | 466 |

### 1. DNA extraction and Quantification of DNA concentration

DNA extracted from C. *perfringens strains isolated* from intestinal contents was performed with commercial DNA extraction kits from Qiagen, Hilden, Germany, according to the manufacturer's instruction.

### 2. DNA amplification

PCR amplification was performed in a final volume of 25µl containing 1 ng of DNA template, 0.2µM of each of the primers for the gene to be amplified or detected and 12 µl of 1 x PCR master mix consisting of 1.2 units/25 µL of Taq DNA polymerase. The amplification was carried out in a Gene amplification PCR system, 9600 Thermocycler (Master cycler eppendorf, Germany). Initial denaturation was at 95°C for 5 min., followed by 40 cycles for 95°C for 30 sec., annealing at 55°C for 30 sec and extension at 72°C for 30 sec, with a final extension at 72°C for 7 minutes and hold for 40°C.

### Sample processing

A total of 36 plasma samples, 96 fecal samples and 87 cecal samples were processed for isolation and extraction of microvesicle total RNA. Plasma volumes varied between 0.5 ml to 3 ml from the different animals.

### Plasma preparation

Briefly, blood was collected into K2 EDTA tubes and mixed well with complete inversions. Then the tubes were centrifuged at 1300 x g for 10 min to separate the blood cells and plasma. Then plasma was removed and filtered through a 0.8µm filter to remove cell debris and platelets. All plasma samples were then aliquoted into 1ml cryovials and stored at -80°C until use.

### Isolation of Microvesicles and RNA extraction

State of the art methods of extracting microvesicles and further purification of the contents of microvesicles (MV) from biomaterials such as blood, csf, tissues and cell cultures have been described previously in US6899863B1 (Dhellin et al., 2005), EP 2 495 025 A1, WO 2014107571 A and 20140178885A1. Methods include the use of high speed ultracentrifugation, or the use of low speed centrifugation to separate cell debris followed by size exclusion or affinity binding to specific membranes or filtration (see review by Witwer & Colleagues, 2013). Several Immunological methods of purifying microvesicles have also been described (Thery, et al., 2016; Clayton et al., 2001; Wubbolts et al., 2003). Microvesicles can be purified by the use of matrixes, beads or magnetic particles functionalized with antibodies binding to surface proteins of microvesicles to positively select a desired population or with antibodies binding to surface proteins of other cellular components for their depletion (Kim et al., 2012 and Mathivanan et al., 2010). Rapid methods of isolation of microvisicles available to research include the use of microfluidic devices (2010, Chen, et al., 2010; EP 2740536 A2) and commercial kits (e.g., SeraMir™ (System Biosciences), ExoSpin™ (Cell Guidance), ExoMir™ (Bioo Scientifics), miRCURY™ (Exiqon), requiring little or no specialized laboratory equipment.

Herein, extraction of microvesicles from plasma, fecal and cecal samples was performed by the methods described in WO2014/107571A1 (Enderle, et al., 2014). The method involved the isolation of microvesicles from a biological fluid by capturing them on the surface of a Q functionalized membrane filter (Exo50, Exosome Diagnostics). The RNA content of the captured microvesicles was released by lysis with Qiazol, and then the RNA was purified by chloroform extraction or with commercial RNAesay kit from Qiagen. In brief, the plasma sample was allowed to thaw slowly on ice and diluted 1:1 with PBS or 0.9% NaOH; the plasma solution was centrifuged at 300 g for 10 minutes to remove cells and debris. The supernatant was passed through a 0.8µm filter and then the flow through was centrifuged at 20,000 g at 4°C for 30 minutes to remove any remaining cell debris. Exo50 column with affinity membrane that binds microvesicles was pre-conditioned by allowing 5 ml loading buffer (2X) 100 mM Phosphate Buffer, 370mM NaCl) to run through it. Then, the supernatant containing the microvesicles obtained from the second centrifugation step was spiked 1000 copies of Qbeta particle to examine the extraction and recovery efficiency upon loading onto the preconditioned spin column. The column was spun briefly and the flow-through was discarded. The column was washed twice with 2 ml Wash buffer (250mM Bis Tris Propane, pH6.5-7 and (IX) 50mM phosphate buffer, 185mM NaCl) and after the second wash, the column was spun briefly and the flow-through was discarded. Microvesicles bound to the membrane were lysed to release content by the addition of 5ml (2,25 ml) of lysis buffer (Qiazol) and spun to collect the lysate. RNA was extracted from the lysate with RNeasy kit, Qiagen, following the manufacture's instruction. The RNA eluted with RNase-free water was pooled for duplicated samples to obtain a total volume of 46µl per sample. 1 µL RNA was applied on Agilent RNA 6000 Nano Kit to assess the quality and concentration of the RNA, while the remaining RNA was stored at -80°C for further downstream use (QPCR or Sequencing). The extraction efficiency and quantity of RNA extraction was assessed by comparing the Q-beta control target gene (Q-beta coat protein gene) expression in the extracted RNA by RT-PCR analysis to those of the target genes of interest. A standard curve of at least 2, 3, or 4 known concentrations in 10-fold dilution of a control target gene was used to determine the copy number. The copy number detected and the quantity of Q-beta particle added was compared to determine the quality of the isolation and/or extraction process.

### Fecal & cecal Samples

Since plasma and cecal samples contain large coarse materials, additional preprocessing steps are applied before performing microvesicle isolation and RNA extraction using the Exo50 kit and associated methods as described above for plasma samples. For instance, a 200 mg of fecal sample was allowed to thaw slowly; then 1 ml of cold PBS or 0.9% sterile NaOH was added to homogenize the fecal / cecal sample followed by vortexing for about 1 minute. The resulting suspension is diluted 1:1 with PBS and then centrifuged at 20,000g for 30 min at 4° C to remove large unwanted particles. Supernatant was filtered, through a 0.8 µm mixed cellulose ester filter (e.g., Millipore filter) and the filtrate was stored at 4°C until further use or processed as described for plasma sample above.

**Table 2. List of primers and probe used for the qPCR to quantify levels of expression of targets. The miRNA were purchased from Life Technologies and in house designed primers and probe were synthesized by IDT (Integrated DNA Technologies, USA)**

| **Target** | **Assay ID / Primers and probes (where applicable)** |
|---|---|
| miR-155 | TM:000479 (Life Technologies #4427975) |
| miR-16 | TM:000391 (Life Technologies #4427975) |
| miR-24 | TM:000402 (Life Technologies #4427975) |
| miR-206 | TM:000510 (Life Technologies #4427975) |
| virX (IDT) | Forward: 5'- TCGTATTCACTGTAGGAGAACAAG -3' |
| | Reverse: 5'- TGAGCTTTTCTTGCTTTTCTGC -3' |
| | Probe: 5'- /56-FAM/ ACGATCCAG /ZEN/ |
| | TTAGATGCCCAGCTTG/3IABKFQ/ -3' |
| NetB (IDT) | Forward: 5'- TGGTGCTGGAATAAATGCTTCA -3' |
| | Reverse: 5'- ACCGTCCTTAGTCTCAACAA -3' |
| | Probe: 5'- /56-FAM/ TGATGCAAA /ZEN/ |
| | TTTAGCATCATGGGA/3IABkFQ/ -3' |
| SAM (IDT) SybrGreen assay | Forward: 5'- AAATAAGGAGTTGATAAACATGAAA -3' |
| | Reverse: 5'- TGTTACTGGTGAACAACAAAAAT -3' |
| colA (IDT) | Forward: 5'- TGAAAGAACACCAGAGGAAAGT -3' |
| | Reverse: 5'- CCTGCAAAGAACTCTGCTGT -3' |
| | Probe: 5'- /56-FAM/ TGATGCAAA /ZEN/ GTGGGGGCAAGGA |
| | /3IABkFQ/ -3' |
| CPE0956 (IDT) SybrGreen assay | Forward: 5'- AATAGGATTTATAGCTGGAT -3' |
| | Reverse: 5'- AGTTAGTTTTCCAAGGATAG -3' |
| scRNA (IDT) SybrGreen assay | Forward: 5'- TATGTAAGTGGTGTTGAGAG -3' |
| | Reverse: 5'- ATGAAAATGACTGCTTATC -3' |

### QRT-PCR of fecal / cecal derived RNA

Reverse transcription of the fecal and cecal RNA was performed with the SuperScript VILO cDNA Synthesis Kit following the instructions of the manufacturer and the following modifications: the RT reaction consisted of 11µl (5x VILO Reaction Mix), 5.5µl (10x SS Enzyme Mix), 5.5u1 (Nuclease free H20) and 33µl (RNA). While the RT cycle profile consisted of 4 sequential incubation cycles / holding at 25° for 10 min, 42° for 60 min, 85° for 5 min and a final holding step at 4° C.

### Q RT-PCR for Plasma and fecal/cecal derived miRNA:

Reverse transcription of the miRNA was performed in triplicate for each sample by using Reverse Transcription of RNA using TaqMan MicroRNA Reverse Transcription Kit with the following RT master mix recipe and cycling profile: RT reaction consisted of 6µl (RT primer pool), 0.3µl (dNTPs of 100mM), 1.5µl (10x RT buffer), 0.2µl RNase inhibitor, 3.0µl MultiScribe RT and 4µl (4 µl RNA for Fecal and cecal samples, and 2µl RNA + 2µl TE buffer for plasma samples). While the RT cycle profile consisted of 4 sequential incubation cycles / holdings at 16° for 30 min, 42° for 30 min, 85° for 5 min and a final holding step at 4° . The RT primer pool was derived by 10 microliter of each RT primer was pooled and diluted to 1 ml with TE buffer, making the final concentration of each RT primer 0.05X. Five times (5×) of the customized miRNA assay (miR-16, miR-21, miR-24, miR- 155 and miR-206) was added to the respective RT primer pool.

### QPCR of fecal / cecal RNA

qPCR was performed for probe-based assays for RNA genes using the Qiagen Rotor -Gene Q, according to manufacturer's instruction. The annealing temperature of virX, colA and netB assays was 57°C, while the annealing temperature for the analysis of 16s rRNA of *Clostridium perfringens* was 60°C. The master mix reactions for the assays consisted of 5.5u1 (Nuclease free water), 10 µl (TaqMan Fast Universal PCR Master Mix no AmpErase UNG), 0.5µl forward primer (20uM), 1.5 µl reverse primer (20µM), 0.25µl probe (20µM), 0.25µl UNG(Uracil-N-glycosylase), and 2µl cDNA. For the amplification of 16s RNA, 1µl each of forward and reverse was used.

Rotor-Gene SYBR Green PCR Kit was performed for small non coding RNA VR-RNA, e45nt and scRNA. The master mix reactions for the assays consisted of 5.75µl (Nuclease free water), 10 µl (Rotor Gene SYBR 2x Master Mix), 0.5µl forward primer (20uM), 1.5 µl reverse primer (20µM), 0.25µl probe (20µM), 0.25µl UNG (Uracil-N-glycosylase), and 2µl cDNA. The amplification was performed with the following condition: First holding step at 50° C for 2 min, followed by another hold at 95° C for 10 and then 40 cycles consisting of 95° C for 2 seconds, 60° for 15 secs 72o C for 20 secs.

### Statistical analysis

All statistical calculations were performed using the statistics software R. As a result of assay's stoichiometry and overall efficiency of the analytical protocols, replacement of missing values (no signal) were imputed before normalization with 40, which is the highest possible CT value in a qPCR with 40 amplification cycles. Moderate statistics for pairwise comparison of the treatment groups were calculated with the ImFit function in the package limma (limma: linear models for microarray data, Smyth G.K. In: Bioinformatics and Computational Biology Solutions using R and Bioconductor, Springer, New York, pp 397-420, R package version 2.16.5) and the resulting p values were adjusted according to the method of Benjamini and Hochberg, (1995) to obtain the q values (false discovery rate)

### Multivariate Analysis (ANOVA)

The dependency between time, treatment and sample type was qualitatively examined. A formal quantitative assessment of possible associations was performed by running two-way ANOVA and non-parametric Kruskal-Wallis tests. Both methods have been applied independently to miRNA and bacterial RNA data. The two way ANOVA was used to test for two main effects and an interaction. The first main effect was time after treatment (in hours), the second main effect was the type of treatment (control, sham, high dose, low dose). The Kruskal-Wallis test was applied separately to time and treatment, to test for an association of these factors with gene expression. No multivariable interaction was assessed by this method. Three samples types (cecal, excreta and plasma) were analyzed for 4 miRNAs (miR-16, miR-24, miR-155 and miR-206) and a total of 216 duplicate data was available. Also six bacterial targets were analyzed in two sample types (cecal and excreta) and a total of 108 duplicate data was available. The means of the duplicate data (mean Ct value of each sample) were used for further statistical analyses. Analysis was performed on both raw data and normalized data. MiR-191 was used as normalizer for the microRNA analysis, whereas Ct values of 16S rRNA of C. *perfringens* were used as normarlizer for the bacterial targets. The anova lineal model (Anova (lm(expression-time+treatment+time*treatment)) generated p values for three factors (time-effect, treatment-effect, interaction) for the normalized and raw data, while the 2 p values (time effect and treatment effect) were generated from the analyses with Kruskal-Wallis test for the normalized and raw data.

### Results:

**Table 3. Number of dead birds per group after C. perfringens challenge. One bird was dead in each of the groups: low-dose gavage group, the sham group, and the negative control group. 4 birds were dead in the group of birds challenged with high dose of Clostridium perfringens.**

| **Group** | **Initial group size** | **Birds sacrificed for bleeding days 14 & 15** | **Birds lost after C. perfringens inoculation** | **AFCR* - after challenge** | **Percent with typical gross pathology** | **No signs of performance loss and disease** |
|---|---|---|---|---|---|---|
| High-dose gavage | 180 | 54 | 4 | 1.60 | 50.70 | 49.30% |
| Low-dose gavage | 180 | 54 | 1 | 1.60 | 42.12 | 57.88% |
| Sham | 180 | 54 | 1 | 1.57 | 6.06^{b} | 93.94% |
| Negative control | 180 | 54 | 1 | 1.56 | 3.13 | 96.87% |
| P value | | | 0.200 | 0.746 | <0.001 | |

| | | | | | | |
|---|---|---|---|---|---|---|
| * AFCR (adjusted feed conversion ratio) determined as the amount of feed consumed divided by the birds body weight, corrected for mortalities and birds removed due to weight diversion from weight of birds before challenge | | | | | | |

**Table 4: Markers with significantly increased occurrence in membrane vesicles of cecal excrements**

| Marker | Raw FDR | Norm FDR |
|---|---|---|
| Sam region | 1.21E-09 | 9,24E-02 |
| Swim zinc finger region | 9,52E-09 | 9,78E-02 |
| scRNA | 3,21E-05 | 7,93E-03 |
| Mir206 | 8,55E-05 | 9,13E-04 |
| virX | 2,95E-04 | 5,93E-03 |
| netB | 1,04E-03 | 1,30E-03 |
| VR.RNA | 2,89E-03 | 3,21 E-05 |
| colA | 6,11E-03 | 3,73E-04 |

**Table 5: Markers with significantly increased occurrence in membrane vesicles of fecal excrements**

| Marker | Raw FDR | Norm FDR |
|---|---|---|
| Sam region | 2,63E-11 | 8,18E-03 |
| scRNA | 8,94E-05 | 8,64E-04 |
| netB | 2,45E-04 | 2,75E-03 |
| virX | 3,24E-03 | 9,78E-02 |
| colA | 5,01E-03 | 8,68E-04 |
| Swim zinc finger region | 5,81 E-02 | 2,26E-03 |
| VR.RNA | 1,36E-01 | 1,03E-04 |

**Table 6: Markers with significantly increased occurrence in membrane vesicles of blood samples**

| Marker | Norm FDR |
|---|---|
| Mir16 | 6,98E-03 |
| Mir155 | 1,56E-02 |
| Mir24 | 9,40E-02 |

### List of references

1. Thery C, Amigorena S, Raposo G, Clayton A (2006). Isolation and characterization of exosomes from cell culture supernatants and biological fluids. Curr Protoc Cell Biol., Chapter 3: Unit 3.22.
2. Clayton A, Court J, Navabi H, Adams M, Mason MD, Hobot JA, et al. (2001). Analysis of antigen presenting cell derived exosomes, based on immuno-magnetic isolation and flow cytometry. J Immunol. Methods . 2001; 247:163-74.
3. Wubbolts R, Leckie RS, Veenhuizen PT, Schwarzmann G, Mobius W, Hoernschemeyer J, et al. (2003). Proteomic and biochemical analyses of human B cell-derived exosomes. Potential implications for their function and multivesicular body formation. J Biol Chem., 278:10963-72.
4. Kim G, Yoo CE, Kim M, Kang HJ, Park D, Lee M, et al, (2012) Noble polymeric surface conjugated with zwitterionic moieties and antibodies for the isolation of exosomes from human serum. Bioconjug. Chem., 23:2114-20.
5. Mathivanan S, Lim JW, Tauro BJ, Ji H, Moritz RL, Simpson RJ (2010). Proteomics analysis of A33 immunoaffinity-purified exosomes released from the human colon tumor cell line LIM1215 reveals a tissue-specific protein signature. Mol. Cell. Proteomics, 9: 197-208.
6. Smyth G.K (2004) linear models for microarray data In: Bioinformatics and Computational Biology Solutions using R and Bioconductor, Springer, New York, pp 397-420
7. Hochberg and Roy (1995) Controlling the false discovery rate: a practical and powerful approach to multiple testing. Journal of the Royal Statistical Soc. Ser B (Methodological), 57:289-300
8. Heikinheimo, A, and Lindstro"m,M, and Korkeala, H. (2004) Enumeration and Isolation of cpe-Positive Clostridium perfringens Spores from Feces. Journal of Clinical Microbiology, 42, 9: 3992-3997
9. Dinh, H., et al., Modulation of microRNAs in two genetically disparate chicken lines showing different necrotic enteritis disease susceptibility. Vet. Immunol. Immunopathol. (2014), http://dx.doi.org/10.1016/j.vetimm.2014.02.003
10. Aade, U.P., and Wankhede, H.J, and Kaldate, K.D (2012). Haematological parameters change in Gallus gallus domesticus infected with cestode parasite. International Multidisciplinary Research Journal, 2(4):13-15
11. Chuku LC et al. (2012) A comparison of the haematological and biochemical indices of broiler and red jungle (hamburgh) fowl (gallus gallus domesticus), Discovery Nature, 2012,1(1),15-18,
12. Wang et al. (2012) Integrated analysis of microRNA expression and mRNA transcriptome in lungs of avian influenza virus infected broilers BMC Genomics 2012, 13:278
13. Jiang et al. (2014) Membrane vesicles of Clostridium perfringens type A strains induceinnate and adaptive immunity. International Journal of Medical Microbiology 304 (2014) 431-443
14. Obana, et al. (2013) Structural Requirement in Clostridium perfringens Collagenase mRNA 5'Leader Sequence for Translational Induction through Small RNA-mRNA Base Pairing Journal of Bacteriology , Volume 195 Number 12 p. 2937-2946
15. Ohtani et al. (2002). Identification of a novel locus that regulates expression of toxin genes in Clostridium perfringens FEMS Microbiology Letters, 209: 113-118
16. Nakamura et al. (1995) Small cytoplasmic RNA (scRNA) gene from Clostridium perfringens can replace the gene for the Bacillus subtilis scRNA in both growth and sporulationMicrobiology (1995), 141: 2965-2975
17. KEYBURN et al. (2010) Association between avian necrotic enteritis and Clostridium perfringens strains expressing NetB toxin Vet. Res. 41:21
18. Shojadoost et al. (2012). The successful experimental induction of necrotic enteritis in chickens by Clostridium perfringens: a critical review Veterinary Research, 43:74
19. Koga et al. (2011) Exosome can prevent RNase from degrading microRNA in feces. J Gastrointest Oncol 2011; 2: 215-222.
20. Saleem, Gulbeena (2013) Identification of biochemical markers for sub-clinical necrotic enteritis in broiler chickens (In Necrotic enteritis, disease induction, predisposing factors and novel biochemical markers in broilers chickens), Chapter 8, PhD thesis, University of Glasgow, UK.
21. Garcia et al. (2009). Experimental infection of commercial layers using a Salmonella enterica serovar Gallinarum strain: Leukogram and serum acute-phase protein concentrations. Revista Brasileira de Ciência Avícola 11, 263-270.
22. Piercy (1979). Acute phase responses to experimental salmonellosis in calves and colibacillosis in chickens: serum iron and caeruloplasmin. Journal of Comparative Pathology 89, 309-319.
23. Witwer et al. (2013) Standardization of sample collection, isolation and analysis methods in extracellular vesicle research Journal of Extracellular Vesicles, 2: 20360
24. Nilsson, et al. (2009) Prostate cancer-derived urine exosomes: a novel approach tobiomarkers for prostate cancer. Br J Cancer, 100:1603-7.
25. Li et al. (2009) Claudin-containing exosomes in the peripheral circulation of women with ovarian cancer. BMC Cancer, 9:244.
26. Shao et al. (2012) Protein typing of circulating microvesicles allows real time monitoring of glioblastoma therapy. Nat Med. , 18:1835-40.
27. Taylor and Gercel-Taylor Taylor (2008) MicroRNA signatures of tumor-derived exosomes as diagnostic biomarkers of ovarian cancer Gynecologic Oncology, 110 : 13-21
28. Cheruvanky et al. (2007) Rapid isolation of urinary exosomal biomarkers using a nanomembrane ultrafiltration concentrator. Am J Physiol Renal Physiol., 292: F1657-61.
29. Miranda et al.(2010) Nucleic acids within urinary exosomes/ microvesicles are potential biomarkers for renal disease. Kidney Int., 78:191-9.
30. Chen et al. (2010) Microfluidic isolation and transcriptome analysis of serum microvesicles. Lab Chip., 10:505-11.
31. Skog et al. (2008). Glioblastoma microvesicles transport RNA and proteins that promote tumour growth and provide diagnostic biomarkers. Nat Cell Biol., 10:1470-6.
32. Moore et al. (2008). Clostridium toxin netB (WO 2008/148166)
33. Dhellin et al. (2005). Method for preparing membrane vesicles (US6,899,863B1)
34. Lamparski et al. (2004) Method of producing membrane vesicles (US6,812,023B1)
35. Abrignani et al. (2007) Method for the preparation of purified HCV RNA by exosome separation (US7,198,923B1)
36. Giorgio (2012) Filter for the removal of micro-vesicles from biological fluids, methods anddevices using such a filter (EP 2495025 A1)
37. Enderle et al (2014) methods for isolating microvesicles (WO2014/107571)
38. Kyung-Hee et al. (2014) Composition and kit for diagnosing breast cancer including polynucleotide within vesicle, and method of diagnosing breast cancer using the same (US 20140178885A1)
39. Min Soak and Jong-Myeon (2014) Microfluidic apparatus and method of enriching target material in biological sample by using the same (EP2740536 A2).

## Claims

1. A method of detecting avian necrotic enteritis, in particular sub-clinical necrotic enteritis, the method comprising isolating microvesicles from an avian sample and subsequently determining the presence and/or level of at least one marker indicative for necrotic enteritis, wherein the presence and/or an increased level of this at least one marker in comparison to a non-infected control is indicative for necrotic enteritis.

2. The method according to claim 1, wherein the marker is selected from the following sequences:
a) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 1,
b) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 3,
c) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 4;
d) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 5;
e) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 7;
f) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 8;
g) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 9;
h) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 16;
i) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40, 60 or 80, consecutive nucleotides of the nucleotide sequence as depicted in SEQ ID NO: 10;
j) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 11;
k) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 12;
l) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 13;
m) polynucleotides (DNAs and RNAs) which are complementary to the sequences according to (a) to (I);
n) polynucleotides (DNAs and RNAs) which are complementary to the sequences according to (m);
o) polynucleotides which comprise the polynucleotides according to (a) to (n).

3. The method according to claim 2 wherein detection of necrotic enteritis is carried out by detecting at least one of the following sequences:
a) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200 consecutive nucleotides of the nucleotide sequence according to position 1 to 630, in particular 420 to 630, of SEQ ID NO: 1,
b) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence according to position 600 to 1000, in particular 670 to 760, of SEQ ID NO: 3,
c) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence according to position 60 to 240, in particular 80 to 220, of SEQ ID NO: 4;
d) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides according to position 1300 to 1800, in particular 1400 to 1700, of SEQ ID NO: 5;
e) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence according to position 1 to 591, in particular 100 to 280 of SEQ ID NO: 7;
f) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence according to position 240 to 540, in particular 300 to 540 of SEQ ID NO: 8;
g) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence according to position 12 to 418, in particular 240 to 400 of SEQ ID NO: 9;
h) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence according to position 673 to 5628 of SEQ ID NO: 16;
i) polynucleotides which have a sequence identity of at least 80 %, preferably at least 85, 90 or 95 %, most preferably 100 %, to a polynucleotide which comprises at least 8 or 10, preferably at least 12, consecutive nucleotides of the nucleotide sequence according to position 14 to 25 of SEQ ID NO: 10;
j) polynucleotides which have a sequence identity of at least 80 %, preferably at least 85, 90 or 95 %, most preferably 100 %, to a polynucleotide which comprises at least 8 or 10, preferably at least 12, 14, 16, 18 or 20, consecutive nucleotides of the nucleotide sequence according to position 44 to 65 of SEQ ID NO: 11;
k) polynucleotides which have a sequence identity of at least 80 %, preferably at least 85, 90 or 95 %, most preferably 100 %, to a polynucleotide which comprises at least 8 or 10, preferably at least 12, 14, 16, 18 or 20, consecutive nucleotides of the nucleotide sequence according to position 3 to 24 of SEQ ID NO: 12;
l) polynucleotides which have a sequence identity of at least 80 %, preferably at least 85, 90 or 95 %, most preferably 100 %, to a polynucleotide which comprises at least 8 or 10, preferably at least 12, 14, 16, 18 or 20, consecutive nucleotides of the nucleotide sequence according to position 45 to 66 of SEQ ID NO: 13,
m) polynucleotides (DNAs and RNAs) which are complementary to the sequences according to (a) to (I);
n) polynucleotides (DNAs and RNAs) which are complementary to the sequences according to (m);
o) polynucleotides which comprise the polynucleotides according to (a) to (n).

4. A method of detecting avian necrotic enteritis, in particular sub-clinical avian necrotic enteritis, wherein detection of necrotic enteritis is carried out by detecting the presence and/or amount of at least one of the following sequences in an avian sample:
a) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 1,
b) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 3,
c) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 4;
d) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 7;
e) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 9;
f) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40, 60 or 80, consecutive nucleotides of the nucleotide sequence as depicted in SEQ ID NO: 10;
g) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 11;
h) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 12;
i) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 13;
j) polynucleotides (DNAs and RNAs) which are complementary to the sequences according to (a) to (i);
k) polynucleotides (DNAs and RNAs) which are complementary to the sequences according to (j);
l) polynucleotides which comprise the polynucleotides according to (a) to (k).

5. A method of detecting an avian bacterial infection, preferably avian necrotic enteritis, in particular sub-clinical necrotic enteritis, the method comprising isolating microvesicles from an avian sample and subsequently determining the presence and/or level of at least one avian marker indicative for the infection, wherein the presence and/or an increased level of this at least one avian marker in comparison to a non-infected control is indicative for the infection and wherein the at least one avian marker is an avian miRNA and is preferably selected from the group consisting of
a) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40, 60 or 80, consecutive nucleotides of the nucleotide sequence as depicted in SEQ ID NO: 10;
b) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 11;
c) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 12;
d) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 13;
e) polynucleotides (DNAs and RNAs) which are complementary to the sequences according to (a) to (d);
f) polynucleotides (DNAs and RNAs) which are complementary to the sequences according to (e);
g) polynucleotides which comprise the polynucleotides according to (a) to (f).

6. The method according to any one of the preceding claims, wherein the avian sample is a bodily fluid, preferably blood, in particular blood plasma or blood serum, or a bodily excrement, preferably a fecal or cecal excrement.

7. A method of detecting avian necrotic enteritis, in particular sub-clinical avian necrotic enteritis, the method comprising determining the presence and/or level of at least one marker indicative for necrotic enteritis in avian fecal excrements, preferably in microvesicles isolated from avian fecal excrements, wherein the presence and/or an increased level of this at least one marker in comparison to a non-infected control is indicative for necrotic enteritis and wherein the marker is preferably selected from
a) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 1,
b) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 3,
c) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence as depicted in SEQ ID NO: 4;
d) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 5;
e) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 7;
f) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 8;
g) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 9;
h) polynucleotides (DNAs and RNAs) which are complementary to the sequences according to (a) to (g);
i) polynucleotides (DNAs and RNAs) which are complementary to the sequences according to (h);
j) polynucleotides which comprise the polynucleotides according to (a) to (i).

8. A method of detecting avian necrotic enteritis, in particular sub-clinical avian necrotic enteritis, the method comprising determining the presence and/or level of at least one marker indicative for necrotic enteritis in avian cecal excrements, in particular in microvesicles isolated from avian cecal excrements, wherein the presence and/or an increased level of this at least one marker in comparison to a non-infected control is indicative for necrotic enteritis and wherein the marker is preferably selected from
a) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence as depicted in SEQ ID NO: 1,
b) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence as depicted in SEQ ID NO: 3,
c) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence as depicted in SEQ ID NO: 4;
d) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence as depicted in SEQ ID NO: 5;
e) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence as depicted in SEQ ID NO: 7;
f) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence as depicted in SEQ ID NO: 8;
g) polynucleotides which have a sequence identity of at least 80 %, preferably at least 85, 90 or 95 %, most preferably 100 %, to a polynucleotide which comprises at least 10, preferably at least 15, 20 or 25, consecutive nucleotides of the nucleotide sequence as depicted in SEQ ID NO: 9;
h) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 13;
i) polynucleotides (DNAs and RNAs) which are complementary to the sequences according to (a) to (h);
j) polynucleotides (DNAs and RNAs) which are complementary to the sequences according to (i);
k) polynucleotides which comprise the polynucleotides according to (a) to (j).

9. A method of detecting avian necrotic enteritis, in particular sub-clinical necrotic enteritis, the method comprising determining the presence and/or level of at least one marker indicative for necrotic enteritis in avian blood, in particular in microvesicles isolated from avian blood, wherein the presence and/or an increased level of this at least one marker in comparison to a non-infected control is indicative for necrotic enteritis and wherein the at least one marker is preferably selected from
a) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40, 60 or 80, consecutive nucleotides of the nucleotide sequence as depicted in SEQ ID NO: 10;
b) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 11;
c) polynucleotides which have a sequence identity of at least 80 %, preferably at least 85, 90 or 95 %, most preferably 100 %, to a polynucleotide which comprises at least 8 or 10, preferably at least 12, 14, 16, 18, 20, 25, 40 or 60, consecutive nucleotides of the nucleotide sequence as depicted in SEQ ID NO: 12;
d) polynucleotides (DNAs and RNAs) which are complementary to the sequences according to (a) to (c);
e) polynucleotides (DNAs and RNAs) which are complementary to the sequences according to (d);
f) polynucleotides which comprise the polynucleotides according to (a) to (e).

10. A method of detecting an avian infection, in particular a sub-clinical avian infection, preferably an avian bacterial infection, in particular a sub-clinical avian bacterial infection, more preferably necrotic enteritis, in particular sub-clinical necrotic enteritis, the method comprising determining the presence and/or amount of at least one marker indicative for the disease in microvesicles isolated from avian excrements, in particular from fecal or cecal excrements, wherein the presence and/or an increased level of this at least one marker in comparison to a non-infected control is indicative for the disease.

11. The method according to any of the preceding claims, wherein the avian sample is a sample of poultry, preferably a sample of chickens, turkeys, ducks, geese, peacocks, pheasants, partridges, guinea fowl, quails, capercaillies, grouse, pigeons, swans, ostriches and parrots, most preferably chickens.

12. A method of treatment comprising performing the method of any one of the preceding claims and administering a therapeutic agent for the treatment of necrotic enteritis infection.

13. Polynucleotides selected from the group consisting of
a) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 1;
b) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 3;
c) polynucleotides which have a sequence identity of at least 80 %, preferably at least 85, 90 or 95 %, most preferably 100 %, to a polynucleotide which comprises at least 10, preferably at least 15, 20, 25, 40, 60, 100, 150, 180 or 200, consecutive nucleotides of the nucleotide sequence as depicted in SEQ ID NO: 7;
d) polynucleotides (DNAs and RNAs) which are complementary to the sequences according to (a) to (c);
e) polynucleotides which comprise the polynucleotides according to (a) to (d).

14. Vector comprising a polynucleotide according to claim 13.

15. Polypeptides selected from the group consisting of
a) polypeptides which have a sequence identity of at least 80 %, preferably at least 85, 90 or 95 %, most preferably 100 %, to a polypeptide which comprises at least 10, preferably at least 15, 20, 25, 40, 60, 100, 150, 180 or 200, in particular all, consecutive amino acids of the amino acid sequence as depicted in SEQ ID NO: 2;
b) polypeptides which have a sequence identity of at least 80 %, preferably at least 85, 90 or 95 %, most preferably 100 %, to a polypeptide which comprises at least 10, preferably at least 15, 20, 25, 40, 60 or 70, in particular all, consecutive amino acids of the amino acid sequence as depicted in SEQ ID NO: 14;
c) polypeptides which have a sequence identity of at least 80 %, preferably at least 85, 90 or 95 %, most preferably 100 %, to a polypeptide which comprises at least 10, preferably at least 15, 20, 25, 40, 60, 100, 150, 180 or 190, in particular all, consecutive amino acids of the amino acid sequence as depicted in SEQ ID NO: 15;
d) polypeptides which comprise the polypeptides according to (a) to (c).


SEQUENCE LISTING

<110>  Evonik Degussa GmbH

<120>  Method of determining avian necrotic enteritis

<130>  201500092

<160>  17

<170>  PatentIn version 3.5

<210>  1
<211>  1884
<212>  DNA
<213>  Clostridium perfringens

<220>
<221>  CDS
<222>  (631)..(1884)
<223>  SAM gene

<400>  1
cataaaaaat cactcctttt atatttttaa aaacattatc acttttttat taattatttt      60

caatataatt agtataactg gtcaaaaatt atacataatt ggactgcagc acttatctta     120

tatttaatac caagtttatc ttcttctatt atattaaaaa tattttctat ataattatat     180

ttattattta cttaagtatt tagtaaatga attactatac ttctcttcat gattaagctt     240

tatttaaatt tttatatata ttaattaact aaatttaata ctttatttta ctaaaaaaat     300

tatatttgta tttaaataaa aaactactat tttttagtaa aataaagtat ttataaaaat     360

attaatatac atttagttta tttttgatat atcaaaaact tttttatttt taaaaaaagt     420

tgataattta atatagttga attttgctat aatatactaa agtttaaata aggagttgat     480

aaacatgaaa tttaaagttt tagaaaaaaa ctttttaact atgtgtgagc caaatttttg     540

ttgttcacca gtaacaaatt tataaaatat tatactaaaa aaactaactc tgatttcatc     600

agggttagtt ttctatatgg aggttttaaa atg aaa aaa tca atg tat aat att      654
                                 Met Lys Lys Ser Met Tyr Asn Ile
                                 1               5

tat ata caa gga aaa aaa gaa acc gca att ttc aac cta tta aca aga      702
Tyr Ile Gln Gly Lys Lys Glu Thr Ala Ile Phe Asn Leu Leu Thr Arg
    10                  15                  20

aat ata att gtt att tca aac aat gaa tat cta aat ttt gat aaa tta      750
Asn Ile Ile Val Ile Ser Asn Asn Glu Tyr Leu Asn Phe Asp Lys Leu
25                  30                  35                  40

ata aat gat gaa gaa tgt aaa gaa caa aaa tct atg ggt ttt tct tta      798
Ile Asn Asp Glu Glu Cys Lys Glu Gln Lys Ser Met Gly Phe Ser Leu
                45                  50                  55

gaa aat aat att aat gaa att gaa tta atg aaa tat act tta aat aaa      846
Glu Asn Asn Ile Asn Glu Ile Glu Leu Met Lys Tyr Thr Leu Asn Lys
            60                  65                  70

ggt aaa ttt tcc gaa aaa tca atg aca tta ttt tta tca ctc aca aga 894
Gly Lys Phe Ser Glu Lys Ser Met Thr Leu Phe Leu Ser Leu Thr Arg
        75                  80                  85

caa tgt aat tta aat tgc att tat tgc tat caa gat aga cga aaa aca 942
Gln Cys Asn Leu Asn Cys Ile Tyr Cys Tyr Gln Asp Arg Arg Lys Thr
    90                  95                  100

ttt gat gaa gga aat aat ttt tta aat aaa act gct tgg tta aaa att 990
Phe Asp Glu Gly Asn Asn Phe Leu Asn Lys Thr Ala Trp Leu Lys Ile
105                 110                 115                 120

ttt gaa ttt tta aag aaa aaa tct att aat tta aat gaa tta cat ata 1038
Phe Glu Phe Leu Lys Lys Lys Ser Ile Asn Leu Asn Glu Leu His Ile
                125                 130                 135

act tta ttt ggt gga gaa cct tta cta aat aaa tca att att tta caa 1086
Thr Leu Phe Gly Gly Glu Pro Leu Leu Asn Lys Ser Ile Ile Leu Gln
            140                 145                 150

att att gat gat tta aat tct tta aaa aat caa act cta aaa ata tat 1134
Ile Ile Asp Asp Leu Asn Ser Leu Lys Asn Gln Thr Leu Lys Ile Tyr
        155                 160                 165

ata aat tta ata act aat gga gtt ctt tta gat aaa aat ttt tgc gaa 1182
Ile Asn Leu Ile Thr Asn Gly Val Leu Leu Asp Lys Asn Phe Cys Glu
    170                 175                 180

aaa ata tat aaa tct ata aat tat att caa att aca ata gat ggt aat 1230
Lys Ile Tyr Lys Ser Ile Asn Tyr Ile Gln Ile Thr Ile Asp Gly Asn
185                 190                 195                 200

aaa tca act cat aat caa tta aga gtt ttt cca gat ggt tct gga agc 1278
Lys Ser Thr His Asn Gln Leu Arg Val Phe Pro Asp Gly Ser Gly Ser
                205                 210                 215

ttt gat att att tat aat aat tta aag gaa gta att aat cta ttt gaa 1326
Phe Asp Ile Ile Tyr Asn Asn Leu Lys Glu Val Ile Asn Leu Phe Glu
            220                 225                 230

aat aaa ata gaa tta aga att aat gta aat aaa gaa ata ata tta aat 1374
Asn Lys Ile Glu Leu Arg Ile Asn Val Asn Lys Glu Ile Ile Leu Asn
        235                 240                 245

tgc ttt tct tta ttt aaa aaa tta aag gaa gat tct tta aat aaa agt 1422
Cys Phe Ser Leu Phe Lys Lys Leu Lys Glu Asp Ser Leu Asn Lys Ser
    250                 255                 260

ata gcc ata aat tta cat cca att ttt gaa aat caa agc tct ttc gaa 1470
Ile Ala Ile Asn Leu His Pro Ile Phe Glu Asn Gln Ser Ser Phe Glu
265                 270                 275                 280

tat ggc tgt aaa aag aat aca gac aaa gac ctt ata tta caa att aat 1518
Tyr Gly Cys Lys Lys Asn Thr Asp Lys Asp Leu Ile Leu Gln Ile Asn
                285                 290                 295

gat tta tat aat tat tta tct aaa cat aat ttt aaa ttt aga aaa aac 1566
Asp Leu Tyr Asn Tyr Leu Ser Lys His Asn Phe Lys Phe Arg Lys Asn
            300                 305                 310

ttc ata gag ggt cca tgt ata gct aaa cat ata aat agt ttt gct ata 1614
Phe Ile Glu Gly Pro Cys Ile Ala Lys His Ile Asn Ser Phe Ala Ile

315                 320                 325

gat gaa aat cta aat att tat aaa tgt cca ggc ttt tta tat tct aag        1662
Asp Glu Asn Leu Asn Ile Tyr Lys Cys Pro Gly Phe Leu Tyr Ser Lys
    330                 335                 340

tct aat gga ttt ata aat gat att gga gaa ata aac ttt tta gac tct        1710
Ser Asn Gly Phe Ile Asn Asp Ile Gly Glu Ile Asn Phe Leu Asp Ser
345                 350                 355                 360

gat ttc ttt aaa gaa gta aat tct gaa caa aaa tgt att aat tct tgt        1758
Asp Phe Phe Lys Glu Val Asn Ser Glu Gln Lys Cys Ile Asn Ser Cys
                365                 370                 375

ata tat gct cct att tgt tat ggt gga tgt act tgg caa aat aaa tgt        1806
Ile Tyr Ala Pro Ile Cys Tyr Gly Gly Cys Thr Trp Gln Asn Lys Cys
            380                 385                 390

aat aaa gag att tta gat tta act tta gaa tca caa tta aaa tct tat        1854
Asn Lys Glu Ile Leu Asp Leu Thr Leu Glu Ser Gln Leu Lys Ser Tyr
        395                 400                 405

tta ata tct aga tat aat ttt gaa ata taa                                1884
Leu Ile Ser Arg Tyr Asn Phe Glu Ile
    410                 415


<210>  2
<211>  417
<212>  PRT
<213>  Clostridium perfringens

<400>  2

Met Lys Lys Ser Met Tyr Asn Ile Tyr Ile Gln Gly Lys Lys Glu Thr
1               5                   10                  15


Ala Ile Phe Asn Leu Leu Thr Arg Asn Ile Ile Val Ile Ser Asn Asn
            20                  25                  30


Glu Tyr Leu Asn Phe Asp Lys Leu Ile Asn Asp Glu Glu Cys Lys Glu
        35                  40                  45


Gln Lys Ser Met Gly Phe Ser Leu Glu Asn Asn Ile Asn Glu Ile Glu
    50                  55                  60


Leu Met Lys Tyr Thr Leu Asn Lys Gly Lys Phe Ser Glu Lys Ser Met
65                  70                  75                  80


Thr Leu Phe Leu Ser Leu Thr Arg Gln Cys Asn Leu Asn Cys Ile Tyr
                85                  90                  95


Cys Tyr Gln Asp Arg Arg Lys Thr Phe Asp Glu Gly Asn Asn Phe Leu
            100                 105                 110


Asn Lys Thr Ala Trp Leu Lys Ile Phe Glu Phe Leu Lys Lys Lys Ser

115                 120                 125

Ile Asn Leu Asn Glu Leu His Ile Thr Leu Phe Gly Gly Glu Pro Leu
    130                 135                 140

Leu Asn Lys Ser Ile Ile Leu Gln Ile Ile Asp Asp Leu Asn Ser Leu
145                 150                 155                 160

Lys Asn Gln Thr Leu Lys Ile Tyr Ile Asn Leu Ile Thr Asn Gly Val
                165                 170                 175

Leu Leu Asp Lys Asn Phe Cys Glu Lys Ile Tyr Lys Ser Ile Asn Tyr
            180                 185                 190

Ile Gln Ile Thr Ile Asp Gly Asn Lys Ser Thr His Asn Gln Leu Arg
        195                 200                 205

Val Phe Pro Asp Gly Ser Gly Ser Phe Asp Ile Ile Tyr Asn Asn Leu
    210                 215                 220

Lys Glu Val Ile Asn Leu Phe Glu Asn Lys Ile Glu Leu Arg Ile Asn
225                 230                 235                 240

Val Asn Lys Glu Ile Ile Leu Asn Cys Phe Ser Leu Phe Lys Lys Leu
                245                 250                 255

Lys Glu Asp Ser Leu Asn Lys Ser Ile Ala Ile Asn Leu His Pro Ile
            260                 265                 270

Phe Glu Asn Gln Ser Ser Phe Glu Tyr Gly Cys Lys Lys Asn Thr Asp
        275                 280                 285

Lys Asp Leu Ile Leu Gln Ile Asn Asp Leu Tyr Asn Tyr Leu Ser Lys
    290                 295                 300

His Asn Phe Lys Phe Arg Lys Asn Phe Ile Glu Gly Pro Cys Ile Ala
305                 310                 315                 320

Lys His Ile Asn Ser Phe Ala Ile Asp Glu Asn Leu Asn Ile Tyr Lys
                325                 330                 335

Cys Pro Gly Phe Leu Tyr Ser Lys Ser Asn Gly Phe Ile Asn Asp Ile
            340                 345                 350

Gly Glu Ile Asn Phe Leu Asp Ser Asp Phe Phe Lys Glu Val Asn Ser
        355                 360                 365

Glu Gln Lys Cys Ile Asn Ser Cys Ile Tyr Ala Pro Ile Cys Tyr Gly
    370                 375                 380

Gly Cys Thr Trp Gln Asn Lys Cys Asn Lys Glu Ile Leu Asp Leu Thr
385                 390                 395                 400

Leu Glu Ser Gln Leu Lys Ser Tyr Leu Ile Ser Arg Tyr Asn Phe Glu
                405                 410                 415

Ile

<210>  3
<211>  1679
<212>  DNA
<213>  Clostridium perfringens

<400>  3
ttaaaaatag gttctaacaa ccatttctgt aaacatacca gtaggatttt ttctttggct      60

taaaaagtat tcctttttat ctttaatctt ttcttttgac catctcatat aatcactctc      120

ctaaaatatt atggttatta taataaaaag aaagtatcta aaatagatac tttcttagtt      180

ataagttgaa atgtgaaaag ctataattat gatataaaaa ctcttaaaag atacacctta      240

attataactt taaatttaat aaataaacag tatattactc tttatttttc ttaaaaaaat      300

ttagtattgc atttttatct tttctacgtg gaaatgttaa atctaagaac tccatcattt      360

tctttttagt ttgggttcat cctagtattg aagtattctt tagaatattt cctctctttt      420

tgaaaatttc atcttcagta tctctaaatc ttgctttttc tgcagcattt attatacccg      480

cagccatttt tatattatcc tggatttgtt tctcgtttaa tgaaccgtta aaattcctaa      540

attcaactgt aggagctcta tctcttgcta tattagttaa gttaatacca taatatataa      600

tttccttcag aaatttttgc tgccctcctt cttacatcct catcattatc catgttgaat      660

cggatatatt tactttcatc ggccctagga ctaaatggag tggcataatg tctggcatta      720

cttcttactc ttcctaaatc tccacctgca gctctataaa tacaatcctc atatacttct      780

atagtcttaa aaaatcttct ccatctttgt cttgcagtat ctagtttatc catgtttata      840

tgaacatggc caccgcacct ttgatctact ctagccccat gtcttttagc aacatcacat      900

attattttta tttgttccca cgtttctgga gtatctttaa gaatagggga aactatttct      960

ccgctggcat ctccatcgga tacggaccca tctctctcga gtttccactt acctggctca     1020

cttcttgaat gataacttaa tcttctagga gagcttacta tacctagctc ataaagttct     1080

atggctatag cattaacatc accaccaaca aactcaagtt caactccgaa tgtactattg     1140

tttccgttta gtacgttttc gtactcatat tctatagaat cgtcattaat ttctgcaaaa     1200

tcgttattga aatcagcttc attgtctgta taaaagtgat tatcgtcttg ttcttcccca     1260

cgaatatttc tatttatttc atctcttata tttctagctc ttctagtagc ttctacattg 1320

tttatagtat ctacttctgc aacttcgcct aaagcttttc tgaccgctat taagtgtctg 1380

cagcattcat ttctataaac atatttgcca cattcacagc tattttcatt catatcaaca 1440

atgtaagttc cttcttcagt tgaacttact tcatatcgat tattacccaa ggaaactata 1500

tttatatctt caattctgtt tctggttcta gataaccctt gaacagttac aatgttatct 1560

aatctaccat taacagcatt aactaaatct tctattattc cttcagaaag attagatcct 1620

gagccatcaa ttaatccttc cgttgtattt gcaatagtat tgcttcttct tttaggcat 1679

<210> 4
<211> 268
<212> DNA
<213> Clostridium perfringens

<400> 4
gccgtgctag atggggagtt agcggtgccc tgtaacctgc aatccgctac agcaggatcg 60

aagtcctcat agaggctcta acttgtggag tctgccctat gtaagtggtg ttgagagttg 120

ggccccacgc aatggaaacc tgtgaacctc gtcaggtccg gaaggaagca gcgataagca 180

gtcattttca tgtgccgtgg atacgtctgg cttgagctaa ctgcataggt aacgttcata 240

agttattgtc gaagtgaggt gcacggtt 268

<210> 5
<211> 3315
<212> DNA
<213> Clostridium perfringens

<220>
<221> CDS
<222> (1)..(3315)
<223> ColA gene

<400> 5
atg aag aaa aac tta aaa agg gga gag cta aca aaa ctg aag tta gtt 48
Met Lys Lys Asn Leu Lys Arg Gly Glu Leu Thr Lys Leu Lys Leu Val
1               5                   10                  15

gaa aga tgg tca gct acc ttt act tta gca gca ttt att tta ttt aat 96
Glu Arg Trp Ser Ala Thr Phe Thr Leu Ala Ala Phe Ile Leu Phe Asn
            20                  25                  30

agc tca ttt aaa gta ttt gca gct gat aaa aag gta gag aat agt aat 144
Ser Ser Phe Lys Val Phe Ala Ala Asp Lys Lys Val Glu Asn Ser Asn
        35                  40                  45

aat gga cag att act aga gag att aat gct gat cag att tct aaa aca 192
Asn Gly Gln Ile Thr Arg Glu Ile Asn Ala Asp Gln Ile Ser Lys Thr
    50                  55                  60

gaa tta aat aat gag gta gct aca gac aat aat aga cca tta gga cct 240
Glu Leu Asn Asn Glu Val Ala Thr Asp Asn Asn Arg Pro Leu Gly Pro

65                  70                  75                  80

agt att gct cca tca aga gca aga aac aac aag atc tat aca ttc gat      288
Ser Ile Ala Pro Ser Arg Ala Arg Asn Asn Lys Ile Tyr Thr Phe Asp
                85                  90                  95

gaa ctt aac aga atg aat tat agt gat cta gtt gaa tta ata aaa aca      336
Glu Leu Asn Arg Met Asn Tyr Ser Asp Leu Val Glu Leu Ile Lys Thr
            100                 105                 110

ata agt tat gaa aac gta cca gac tta ttt aat ttt aat gat ggt tca      384
Ile Ser Tyr Glu Asn Val Pro Asp Leu Phe Asn Phe Asn Asp Gly Ser
        115                 120                 125

tat act ttc ttt agt aat aga gat cgt gta caa gct ata ata tat ggt      432
Tyr Thr Phe Phe Ser Asn Arg Asp Arg Val Gln Ala Ile Ile Tyr Gly
    130                 135                 140

cta gag gat agt gga aga act tat aca gca gat gat gat aag gga att      480
Leu Glu Asp Ser Gly Arg Thr Tyr Thr Ala Asp Asp Asp Lys Gly Ile
145                 150                 155                 160

cca act tta gtt gag ttt tta aga gct gga tat tat tta gga ttt tat      528
Pro Thr Leu Val Glu Phe Leu Arg Ala Gly Tyr Tyr Leu Gly Phe Tyr
                165                 170                 175

aat aaa caa tta tca tac cta aat aca cca cag tta aaa aat gag tgt      576
Asn Lys Gln Leu Ser Tyr Leu Asn Thr Pro Gln Leu Lys Asn Glu Cys
            180                 185                 190

tta cca gct atg aaa gcg att caa tat aat agt aat ttt aga tta gga      624
Leu Pro Ala Met Lys Ala Ile Gln Tyr Asn Ser Asn Phe Arg Leu Gly
        195                 200                 205

aca aag gcg caa gat gga gtt gtt gag gct tta gga aga ctt ata ggt      672
Thr Lys Ala Gln Asp Gly Val Val Glu Ala Leu Gly Arg Leu Ile Gly
    210                 215                 220

aat gct tca gca gat cca gaa gtt att aat aat tgc ata tat gtt tta      720
Asn Ala Ser Ala Asp Pro Glu Val Ile Asn Asn Cys Ile Tyr Val Leu
225                 230                 235                 240

agt gat ttt aaa gat aat ata gat aag tat ggt tct aac tat agc aag      768
Ser Asp Phe Lys Asp Asn Ile Asp Lys Tyr Gly Ser Asn Tyr Ser Lys
                245                 250                 255

gga aat gca gta ttc aac ctt atg aaa ggt att gat tat tat act aat      816
Gly Asn Ala Val Phe Asn Leu Met Lys Gly Ile Asp Tyr Tyr Thr Asn
            260                 265                 270

tca gta ata tac aat act aag gga tat gat gct aaa aac act gag ttc      864
Ser Val Ile Tyr Asn Thr Lys Gly Tyr Asp Ala Lys Asn Thr Glu Phe
        275                 280                 285

tat aat aga ata gat cca tat atg gaa aga tta gaa agt tta tgt aca      912
Tyr Asn Arg Ile Asp Pro Tyr Met Glu Arg Leu Glu Ser Leu Cys Thr
    290                 295                 300

ata ggt gat aag tta aat aat gat aat gct tgg ctt gta aat aat gcc      960
Ile Gly Asp Lys Leu Asn Asn Asp Asn Ala Trp Leu Val Asn Asn Ala
305                 310                 315                 320

tta tat tac aca ggt aga atg ggt aag ttt aga gaa gac cca tca ata     1008

Leu Tyr Tyr Thr Gly Arg Met Gly Lys Phe Arg Glu Asp Pro Ser Ile
                325                 330                 335

tct caa aga gct tta gaa aga gct atg aag gag tat cct tat tta tca      1056
Ser Gln Arg Ala Leu Glu Arg Ala Met Lys Glu Tyr Pro Tyr Leu Ser
            340                 345                 350

tat caa tat att gaa gct gcc aat gat tta gat tta aat ttt ggt ggc      1104
Tyr Gln Tyr Ile Glu Ala Ala Asn Asp Leu Asp Leu Asn Phe Gly Gly
        355                 360                 365

aaa aat tca tca gga aat gat ata gat ttc aat aag ata aaa gca gat      1152
Lys Asn Ser Ser Gly Asn Asp Ile Asp Phe Asn Lys Ile Lys Ala Asp
    370                 375                 380

gca agg gaa aaa tat ctt cca aaa aca tat act ttt gat gat ggt aaa      1200
Ala Arg Glu Lys Tyr Leu Pro Lys Thr Tyr Thr Phe Asp Asp Gly Lys
385                 390                 395                 400

ttt gta gta aaa gct ggt gat aaa gta aca gaa gag aag ata aaa aga      1248
Phe Val Val Lys Ala Gly Asp Lys Val Thr Glu Glu Lys Ile Lys Arg
                405                 410                 415

tta tat tgg gct tca aag gaa gtt aag gct caa ttc atg aga gta gtt      1296
Leu Tyr Trp Ala Ser Lys Glu Val Lys Ala Gln Phe Met Arg Val Val
            420                 425                 430

caa aat gat aag gct tta gaa gag gga aat cca gat gat att tta act      1344
Gln Asn Asp Lys Ala Leu Glu Glu Gly Asn Pro Asp Asp Ile Leu Thr
        435                 440                 445

gtt gtt att tat aac tca cca gaa gag tat aag tta aat cgt ata ata      1392
Val Val Ile Tyr Asn Ser Pro Glu Glu Tyr Lys Leu Asn Arg Ile Ile
    450                 455                 460

aat gga ttt agt act gat aat ggt ggt ata tat att gaa aac ata gga      1440
Asn Gly Phe Ser Thr Asp Asn Gly Gly Ile Tyr Ile Glu Asn Ile Gly
465                 470                 475                 480

act ttc ttt act tat gaa aga aca cca gag gaa agt ata tat aca tta      1488
Thr Phe Phe Thr Tyr Glu Arg Thr Pro Glu Glu Ser Ile Tyr Thr Leu
                485                 490                 495

gaa gaa tta ttc cgt cat gaa ttt act cac tat ctt caa ggt aga tat      1536
Glu Glu Leu Phe Arg His Glu Phe Thr His Tyr Leu Gln Gly Arg Tyr
            500                 505                 510

gta gtt cct gga atg tgg ggg caa gga gaa ttc tat caa gag gga gtt      1584
Val Val Pro Gly Met Trp Gly Gln Gly Glu Phe Tyr Gln Glu Gly Val
        515                 520                 525

tta act tgg tat gaa gaa gga aca gca gag ttc ttt gca ggt tca act      1632
Leu Thr Trp Tyr Glu Glu Gly Thr Ala Glu Phe Phe Ala Gly Ser Thr
    530                 535                 540

aga act gat gga ata aaa cca aga aaa tca gtt aca caa ggg tta gct      1680
Arg Thr Asp Gly Ile Lys Pro Arg Lys Ser Val Thr Gln Gly Leu Ala
545                 550                 555                 560

tac gat aga aat aat aga atg tct tta tat ggt gta tta cat gct aaa      1728
Tyr Asp Arg Asn Asn Arg Met Ser Leu Tyr Gly Val Leu His Ala Lys
                565                 570                 575

tat ggc tca tgg gat ttc tat aat tat gga ttt gct cta tca aac tac 1776
Tyr Gly Ser Trp Asp Phe Tyr Asn Tyr Gly Phe Ala Leu Ser Asn Tyr
            580                 585                 590

atg tac aac aat aac atg gga atg ttt aat aag atg aca aat tac ata 1824
Met Tyr Asn Asn Asn Met Gly Met Phe Asn Lys Met Thr Asn Tyr Ile
        595                 600                 605

aag aat aat gat gta tct ggt tat aaa gat tat att gca tca atg agt 1872
Lys Asn Asn Asp Val Ser Gly Tyr Lys Asp Tyr Ile Ala Ser Met Ser
    610                 615                 620

agt gat tac gga tta aat gat aaa tat caa gac tat atg gat tct tta 1920
Ser Asp Tyr Gly Leu Asn Asp Lys Tyr Gln Asp Tyr Met Asp Ser Leu
625                 630                 635                 640

tta aac aat att gat aac tta gat gtt cct tta gtt tca gat gag tat 1968
Leu Asn Asn Ile Asp Asn Leu Asp Val Pro Leu Val Ser Asp Glu Tyr
                645                 650                 655

gta aat gga cat gaa gct aag gat ata aat gaa ata act aat gac ata 2016
Val Asn Gly His Glu Ala Lys Asp Ile Asn Glu Ile Thr Asn Asp Ile
            660                 665                 670

aaa gaa gtt tca aat ata aaa gat ctt tct agt aat gtt gaa aag tct 2064
Lys Glu Val Ser Asn Ile Lys Asp Leu Ser Ser Asn Val Glu Lys Ser
        675                 680                 685

caa ttc ttt act act tac gat atg aga gga aca tat gta ggg gga aga 2112
Gln Phe Phe Thr Thr Tyr Asp Met Arg Gly Thr Tyr Val Gly Gly Arg
    690                 695                 700

agt caa ggg gaa gaa aat gac tgg aaa gat atg aat tct aag tta aat 2160
Ser Gln Gly Glu Glu Asn Asp Trp Lys Asp Met Asn Ser Lys Leu Asn
705                 710                 715                 720

gat atg tta aaa gaa tta tct aaa aag agc tgg aat ggt tat aaa act 2208
Asp Met Leu Lys Glu Leu Ser Lys Lys Ser Trp Asn Gly Tyr Lys Thr
                725                 730                 735

gtt act gca tac ttt gta aac cat aaa gta gat gaa aat ggt aac tat 2256
Val Thr Ala Tyr Phe Val Asn His Lys Val Asp Glu Asn Gly Asn Tyr
            740                 745                 750

gtt tat gat gtt gta ttc cat gga atg aat aca gat aca aat act gat 2304
Val Tyr Asp Val Val Phe His Gly Met Asn Thr Asp Thr Asn Thr Asp
        755                 760                 765

gtt cat gtt aat aaa gag cct aag gct gtt ata aaa tct gat tct tca 2352
Val His Val Asn Lys Glu Pro Lys Ala Val Ile Lys Ser Asp Ser Ser
    770                 775                 780

gta ata gtt gaa gaa gaa att aat ttt gat gga aca gag tca aag gat 2400
Val Ile Val Glu Glu Glu Ile Asn Phe Asp Gly Thr Glu Ser Lys Asp
785                 790                 795                 800

gaa gat ggt gaa atc aaa gct tat gaa tgg gac ttt gga gat gga gaa 2448
Glu Asp Gly Glu Ile Lys Ala Tyr Glu Trp Asp Phe Gly Asp Gly Glu
                805                 810                 815

aaa tct aat gag gct aaa gcc act cat aag tat aat aaa act gga gaa 2496
Lys Ser Asn Glu Ala Lys Ala Thr His Lys Tyr Asn Lys Thr Gly Glu
            820                 825                 830

tat gaa gta aaa tta aca gtt aca gat aat aac ggt gga ata aat act 2544
Tyr Glu Val Lys Leu Thr Val Thr Asp Asn Asn Gly Gly Ile Asn Thr
        835                 840                 845

gaa agt aaa aag ata aaa gta gta gaa gat aaa cct gtt gaa gtt ata 2592
Glu Ser Lys Lys Ile Lys Val Val Glu Asp Lys Pro Val Glu Val Ile
    850                 855                 860

aat gaa agc gag cca aac aat gat ttt gaa aag gct aac caa ata gct 2640
Asn Glu Ser Glu Pro Asn Asn Asp Phe Glu Lys Ala Asn Gln Ile Ala
865                 870                 875                 880

aaa tct aat atg tta gtt aag ggt act tta tca gaa gag gat tat tca 2688
Lys Ser Asn Met Leu Val Lys Gly Thr Leu Ser Glu Glu Asp Tyr Ser
                885                 890                 895

gat aaa tat tat ttt gat gta gct aaa aaa ggc aat gtt aaa atc act 2736
Asp Lys Tyr Tyr Phe Asp Val Ala Lys Lys Gly Asn Val Lys Ile Thr
            900                 905                 910

ctt aat aat tta aat tca gta gga ata act tgg aca ctt tat aaa gag 2784
Leu Asn Asn Leu Asn Ser Val Gly Ile Thr Trp Thr Leu Tyr Lys Glu
        915                 920                 925

gga gac cta aac aat tat gtt tta tat gca act gga aat gat gga aca 2832
Gly Asp Leu Asn Asn Tyr Val Leu Tyr Ala Thr Gly Asn Asp Gly Thr
    930                 935                 940

gaa tta aag ggt gaa aag act tta gag cct gga aga tac tac tta agt 2880
Glu Leu Lys Gly Glu Lys Thr Leu Glu Pro Gly Arg Tyr Tyr Leu Ser
945                 950                 955                 960

gta tat act tat gat aat caa tca gga gct tac aca gta aat gta aaa 2928
Val Tyr Thr Tyr Asp Asn Gln Ser Gly Ala Tyr Thr Val Asn Val Lys
                965                 970                 975

gga aac ctt aaa aat gaa gtt aaa gaa aca gaa aag gat gct ata aaa 2976
Gly Asn Leu Lys Asn Glu Val Lys Glu Thr Glu Lys Asp Ala Ile Lys
            980                 985                 990

gaa gtt gaa aat aac aat gat ttt gat aaa gct atg aag gta gac agt 3024
Glu Val Glu Asn Asn Asn Asp Phe Asp Lys Ala Met Lys Val Asp Ser
        995                 1000                1005

aat agc aaa ata gtt gga aca tta agc aat gat gat ctt aag gat 3069
Asn Ser Lys Ile Val Gly Thr Leu Ser Asn Asp Asp Leu Lys Asp
    1010                1015                1020

att tat agc ata gat ata caa aat cca agt gac tta aac ata gta 3114
Ile Tyr Ser Ile Asp Ile Gln Asn Pro Ser Asp Leu Asn Ile Val
    1025                1030                1035

gtt gaa aac tta gat aat ata aaa atg aac tgg tta tta tat tca 3159
Val Glu Asn Leu Asp Asn Ile Lys Met Asn Trp Leu Leu Tyr Ser
    1040                1045                1050

gct gat gat tta agt aac tat gtg gat tac gct aat gca gat gga 3204
Ala Asp Asp Leu Ser Asn Tyr Val Asp Tyr Ala Asn Ala Asp Gly
    1055                1060                1065

aat aaa tta agt aac act tgt aag tta aat cca ggt aaa tat tac 3249
Asn Lys Leu Ser Asn Thr Cys Lys Leu Asn Pro Gly Lys Tyr Tyr

1070                    1075                    1080

tta tgt  gtt tat caa ttt gaa  aac tca ggt act gga  aat tac aca         3294
Leu Cys  Val Tyr Gln Phe Glu  Asn Ser Gly Thr Gly  Asn Tyr Thr
    1085                    1090                    1095

gta aac  tta caa aac aaa taa                                           3315
Val Asn  Leu Gln Asn Lys
    1100

<210>  6
<211>  1104
<212>  PRT
<213>  Clostridium perfringens

<400>  6

Met Lys Lys Asn Leu Lys Arg Gly Glu Leu Thr Lys Leu Lys Leu Val
1               5                   10                  15

Glu Arg Trp Ser Ala Thr Phe Thr Leu Ala Ala Phe Ile Leu Phe Asn
            20                  25                  30

Ser Ser Phe Lys Val Phe Ala Ala Asp Lys Lys Val Glu Asn Ser Asn
        35                  40                  45

Asn Gly Gln Ile Thr Arg Glu Ile Asn Ala Asp Gln Ile Ser Lys Thr
    50                  55                  60

Glu Leu Asn Asn Glu Val Ala Thr Asp Asn Asn Arg Pro Leu Gly Pro
65                  70                  75                  80

Ser Ile Ala Pro Ser Arg Ala Arg Asn Asn Lys Ile Tyr Thr Phe Asp
                85                  90                  95

Glu Leu Asn Arg Met Asn Tyr Ser Asp Leu Val Glu Leu Ile Lys Thr
            100                 105                 110

Ile Ser Tyr Glu Asn Val Pro Asp Leu Phe Asn Phe Asn Asp Gly Ser
        115                 120                 125

Tyr Thr Phe Phe Ser Asn Arg Asp Arg Val Gln Ala Ile Ile Tyr Gly
    130                 135                 140

Leu Glu Asp Ser Gly Arg Thr Tyr Thr Ala Asp Asp Asp Lys Gly Ile
145                 150                 155                 160

Pro Thr Leu Val Glu Phe Leu Arg Ala Gly Tyr Tyr Leu Gly Phe Tyr
                165                 170                 175

Asn Lys Gln Leu Ser Tyr Leu Asn Thr Pro Gln Leu Lys Asn Glu Cys

            180                 185                 190

Leu Pro Ala Met Lys Ala Ile Gln Tyr Asn Ser Asn Phe Arg Leu Gly
        195                 200                 205

Thr Lys Ala Gln Asp Gly Val Val Glu Ala Leu Gly Arg Leu Ile Gly
    210                 215                 220

Asn Ala Ser Ala Asp Pro Glu Val Ile Asn Asn Cys Ile Tyr Val Leu
225                 230                 235                 240

Ser Asp Phe Lys Asp Asn Ile Asp Lys Tyr Gly Ser Asn Tyr Ser Lys
                245                 250                 255

Gly Asn Ala Val Phe Asn Leu Met Lys Gly Ile Asp Tyr Tyr Thr Asn
            260                 265                 270

Ser Val Ile Tyr Asn Thr Lys Gly Tyr Asp Ala Lys Asn Thr Glu Phe
        275                 280                 285

Tyr Asn Arg Ile Asp Pro Tyr Met Glu Arg Leu Glu Ser Leu Cys Thr
    290                 295                 300

Ile Gly Asp Lys Leu Asn Asn Asp Asn Ala Trp Leu Val Asn Asn Ala
305                 310                 315                 320

Leu Tyr Tyr Thr Gly Arg Met Gly Lys Phe Arg Glu Asp Pro Ser Ile
                325                 330                 335

Ser Gln Arg Ala Leu Glu Arg Ala Met Lys Glu Tyr Pro Tyr Leu Ser
            340                 345                 350

Tyr Gln Tyr Ile Glu Ala Ala Asn Asp Leu Asp Leu Asn Phe Gly Gly
        355                 360                 365

Lys Asn Ser Ser Gly Asn Asp Ile Asp Phe Asn Lys Ile Lys Ala Asp
    370                 375                 380

Ala Arg Glu Lys Tyr Leu Pro Lys Thr Tyr Thr Phe Asp Asp Gly Lys
385                 390                 395                 400

Phe Val Val Lys Ala Gly Asp Lys Val Thr Glu Glu Lys Ile Lys Arg
                405                 410                 415

Leu Tyr Trp Ala Ser Lys Glu Val Lys Ala Gln Phe Met Arg Val Val
            420                 425                 430

Gln Asn Asp Lys Ala Leu Glu Glu Gly Asn Pro Asp Asp Ile Leu Thr
        435                 440                 445

Val Val Ile Tyr Asn Ser Pro Glu Glu Tyr Lys Leu Asn Arg Ile Ile
    450                 455                 460

Asn Gly Phe Ser Thr Asp Asn Gly Gly Ile Tyr Ile Glu Asn Ile Gly
465                 470                 475                 480

Thr Phe Phe Thr Tyr Glu Arg Thr Pro Glu Glu Ser Ile Tyr Thr Leu
                485                 490                 495

Glu Glu Leu Phe Arg His Glu Phe Thr His Tyr Leu Gln Gly Arg Tyr
            500                 505                 510

Val Val Pro Gly Met Trp Gly Gln Gly Glu Phe Tyr Gln Glu Gly Val
        515                 520                 525

Leu Thr Trp Tyr Glu Glu Gly Thr Ala Glu Phe Phe Ala Gly Ser Thr
    530                 535                 540

Arg Thr Asp Gly Ile Lys Pro Arg Lys Ser Val Thr Gln Gly Leu Ala
545                 550                 555                 560

Tyr Asp Arg Asn Asn Arg Met Ser Leu Tyr Gly Val Leu His Ala Lys
                565                 570                 575

Tyr Gly Ser Trp Asp Phe Tyr Asn Tyr Gly Phe Ala Leu Ser Asn Tyr
            580                 585                 590

Met Tyr Asn Asn Asn Met Gly Met Phe Asn Lys Met Thr Asn Tyr Ile
        595                 600                 605

Lys Asn Asn Asp Val Ser Gly Tyr Lys Asp Tyr Ile Ala Ser Met Ser
    610                 615                 620

Ser Asp Tyr Gly Leu Asn Asp Lys Tyr Gln Asp Tyr Met Asp Ser Leu
625                 630                 635                 640

Leu Asn Asn Ile Asp Asn Leu Asp Val Pro Leu Val Ser Asp Glu Tyr
                645                 650                 655

Val Asn Gly His Glu Ala Lys Asp Ile Asn Glu Ile Thr Asn Asp Ile
            660                 665                 670

Lys Glu Val Ser Asn Ile Lys Asp Leu Ser Ser Asn Val Glu Lys Ser
        675                 680                 685

Gln Phe Phe Thr Thr Tyr Asp Met Arg Gly Thr Tyr Val Gly Gly Arg
    690                 695                 700

Ser Gln Gly Glu Glu Asn Asp Trp Lys Asp Met Asn Ser Lys Leu Asn
705                 710                 715                 720

Asp Met Leu Lys Glu Leu Ser Lys Lys Ser Trp Asn Gly Tyr Lys Thr
                725                 730                 735

Val Thr Ala Tyr Phe Val Asn His Lys Val Asp Glu Asn Gly Asn Tyr
            740                 745                 750

Val Tyr Asp Val Val Phe His Gly Met Asn Thr Asp Thr Asn Thr Asp
        755                 760                 765

Val His Val Asn Lys Glu Pro Lys Ala Val Ile Lys Ser Asp Ser Ser
    770                 775                 780

Val Ile Val Glu Glu Glu Ile Asn Phe Asp Gly Thr Glu Ser Lys Asp
785                 790                 795                 800

Glu Asp Gly Glu Ile Lys Ala Tyr Glu Trp Asp Phe Gly Asp Gly Glu
                805                 810                 815

Lys Ser Asn Glu Ala Lys Ala Thr His Lys Tyr Asn Lys Thr Gly Glu
            820                 825                 830

Tyr Glu Val Lys Leu Thr Val Thr Asp Asn Asn Gly Gly Ile Asn Thr
        835                 840                 845

Glu Ser Lys Lys Ile Lys Val Val Glu Asp Lys Pro Val Glu Val Ile
    850                 855                 860

Asn Glu Ser Glu Pro Asn Asn Asp Phe Glu Lys Ala Asn Gln Ile Ala
865                 870                 875                 880

Lys Ser Asn Met Leu Val Lys Gly Thr Leu Ser Glu Glu Asp Tyr Ser
                885                 890                 895

Asp Lys Tyr Tyr Phe Asp Val Ala Lys Lys Gly Asn Val Lys Ile Thr
            900                 905                 910

Leu Asn Asn Leu Asn Ser Val Gly Ile Thr Trp Thr Leu Tyr Lys Glu
        915                 920                 925

Gly Asp Leu Asn Asn Tyr Val Leu Tyr Ala Thr Gly Asn Asp Gly Thr
    930                 935                 940

Glu Leu Lys Gly Glu Lys Thr Leu Glu Pro Gly Arg Tyr Tyr Leu Ser
945                 950                 955                 960

Val Tyr Thr Tyr Asp Asn Gln Ser Gly Ala Tyr Thr Val Asn Val Lys
                965                 970                 975

Gly Asn Leu Lys Asn Glu Val Lys Glu Thr Glu Lys Asp Ala Ile Lys
            980                 985                 990

Glu Val Glu Asn Asn Asn Asp Phe  Asp Lys Ala Met Lys  Val Asp Ser
        995                 1000                 1005

Asn Ser  Lys Ile Val Gly Thr  Leu Ser Asn Asp Asp  Leu Lys Asp
    1010                 1015                 1020

Ile Tyr  Ser Ile Asp Ile Gln  Asn Pro Ser Asp Leu  Asn Ile Val
    1025                 1030                 1035

Val Glu  Asn Leu Asp Asn Ile  Lys Met Asn Trp Leu  Leu Tyr Ser
    1040                 1045                 1050

Ala Asp  Asp Leu Ser Asn Tyr  Val Asp Tyr Ala Asn  Ala Asp Gly
    1055                 1060                 1065

Asn Lys  Leu Ser Asn Thr Cys  Lys Leu Asn Pro Gly  Lys Tyr Tyr
    1070                 1075                 1080

Leu Cys  Val Tyr Gln Phe Glu  Asn Ser Gly Thr Gly  Asn Tyr Thr
    1085                 1090                 1095

Val Asn  Leu Gln Asn Lys
    1100

<210>  7
<211>  1001
<212>  DNA
<213>  Clostridium perfringens

<400>  7
tcaaaataga gtgcttttac ttataaaact taatatattt attcctaaaa tatcagataa      60

aattaagact atgttatata taatgagaag gaaattaaat atttttagta ttttatttat     120

tatcttttca gtttttgatc cagttttaat tttggctatt ttaaagttag ttttccaagg     180

atagaaaagt tctattcctt ctgaagttaa aaaatctaaa actaagtggg atatatatcc     240

agctataaat cctattgaaa ttattaataa tattatgttg aagttactaa catttaaaat     300

aatatataag gttaatacta ttaaagatag acatagtaaa ctatgagtaa agccacgatg     360

tctacattta ctacctatat gctttgaaag aaaaggatac atttgactaa ttgaagattt 420

tctttgatct atatcaggaa atagagatcc tatataagaa aaatgaaaaa ataatgctat 480

taataaaatt ttataagcta tatcatatgg aaataagaac aggcttaaaa ttgtatttaa 540

cattattaaa gaaatgcata cgcctccagt tgaatgagtt tcttttgtca taaaatctcc 600

tttaaaaata attatcatta gtttatatta aaaatattat cataatattc attttacata 660

aagaagaaaa aaataaaaat cctaaaaata gagtggacag ttctatttct aggatcaata 720

gttatggtgg taattttatg taaggttttt tactaaatta aagtaaaatc attaataagt 780

aattatttat ttttaattac aattatggaa tatgcaattg aaaatattga tattaaagca 840

agtatgggac ttataatatt tgaaacaact tctaaagatg taaaataaga tactctttgt 900

tcaatatata aagctatgct atttttaagt attaagggag aaaaaagagt tattaatgta 960

attaaaattg cacttgttaa aatccttttt gtatgtttca a 1001

<210> 8
<211> 960
<212> DNA
<213> Clostridium perfringens

<220>
<221> misc_feature
<222> (527)..(527)
<223> n is a, c, g, t or u

<400> 8
ttacagataa tattctattt tatgatcttg ccaatttatt ttaaacataa attcgttata 60

ttcacttgtt gacgaaagtt tgtttgttcc tcgccattga gtagtttccc aatttaaaat 120

atagtcatta tcaaatcttt gatattcaac tattattaca gattctttag catttttagg 180

tgctgttaat gctaaagcca tattgggtga aaatccacca gaaattaatg ttgataaatc 240

tctatcatct gtgaaatttt tatcaccatt attatacaat cttgatttca tgaataattg 300

atttccataa atagcatgat aagaatctat attataaccg tccttagtct caacaaattt 360

tatatcccat gatgctaaat ttgcatcatc ttttctttga attgttctaa aatcaggttg 420

ttcatagctt atagtatttt ggacattata tgaagcattt attccagcac cagcagtttt 480

tccttcaaca gatatattac cgcctataga ataaccaatt gaattanata catctttttt 540

atctatagtt ttaggaatag aatttgctat attattattt acatcagcac tttttacatt 600

aattctataa gtttcaggcc atttcatttt tccgtaatat ttagaaccaa aaatctgttt 660

atcagaaggt ataaatcctt ctaaatttaa taaagcagtt ttcttatcag aatgaggatc 720

ttcaataaat gttccactta aagttgcctt ccaaccttta tgtgaagcgg tatcactaga 780

agtatattta atagcttcct ttccattttc ttttattatt tctccactta gatttttcaa 840

ctcaattttg tttatgtcat ttaattcact tgcaaaaact tgagtttgag ttgaaaaaag 900

gcttgtacta attacacttg taagaactag tgtaattgaa ataattttta atcttttcaa 960

<210> 9
<211> 562
<212> DNA
<213> Clostridium perfringens

<400> 9
tgtttatcat cccttttctc tcatttttat atactcataa atatatcata aaaagcaaaa 60

tctatccata atatatttaa aaaatgagta tatagttttt aataaaatta tatttaatta 120

tgtataaaaa tcataagtta aattgctcta tataatattt ccaaataaga ttaattttat 180

gaaatagaaa tgaaaaaagt tctgcaaagt gcagaacttt taatttatga ataaacttgc 240

gttaattatc aattatctat tcattttttg agcttttctt gcttttctgc aagctgggca 300

tctaactgga tcgttttcaa atcctttttc tttgaagaat tcttgttctc ctacagtgaa 360

tacgaattct tttccacagt ctttacaaac taaattttta tcttccattt cttttacctc 420

ctgtaaaatt tactaggtac gtttgagatc tatactataa aactaataaa ataaatttta 480

caggacagaa ataaagtaca tatctactaa attttatttt atatattgat tatatcatat 540

agaaaaaaga atacaatact tt 562

<210> 10
<211> 84
<212> RNA
<213> Gallus gallus

<400> 10
gucugucaua cucuagcagc acguaaauau ugguguuaaa acuguaaaua ucuccaguau 60

uaacugugcu gcugaaguaa ggcu 84

<210> 11
<211> 68
<212> RNA
<213> Gallus gallus

<400> 11
cuccggugcc uacugagcug auaucaguuc ugauuuuaca uacuggcuca guucagcagg 60

aacaggag 68

<210> 12
<211> 63
<212> RNA
<213> Gallus gallus

<400> 12
uguuaaugcu aaucgugaua gggguuuuua ccucugaaug acuccuacau guuagcauua 60

aca                                                                    63

<210>  13
<211>  76
<212>  RNA
<213>  Gallus gallus

<400>  13
gagaugacau gcuucuuuau auccccauau ggauuaggcu gcuauggaau guaaggaagu      60

gugugguuuc agggag                                                      76

<210>  14
<211>  77
<212>  PRT
<213>  Clostridium perfringens

<400>  14

Met Lys Glu Ile Ile Tyr Tyr Gly Ile Asn Leu Thr Asn Ile Ala Arg
1               5                   10                  15

Asp Arg Ala Pro Thr Val Glu Phe Arg Asn Phe Asn Gly Ser Leu Asn
            20                  25                  30

Glu Lys Gln Ile Gln Asp Asn Ile Lys Met Ala Ala Gly Ile Ile Asn
        35                  40                  45

Ala Ala Glu Lys Ala Arg Phe Arg Asp Thr Glu Asp Glu Ile Phe Lys
    50                  55                  60

Lys Arg Gly Asn Ile Leu Lys Asn Thr Ser Ile Leu Gly
65                  70                  75

<210>  15
<211>  196
<212>  PRT
<213>  Clostridium perfringens

<400>  15

Met Thr Lys Glu Thr His Ser Thr Gly Gly Val Cys Ile Ser Leu Ile
1               5                   10                  15

Met Leu Asn Thr Ile Leu Ser Leu Phe Leu Phe Pro Tyr Asp Ile Ala
            20                  25                  30

Tyr Lys Ile Leu Leu Ile Ala Leu Phe Phe His Phe Ser Tyr Ile Gly
        35                  40                  45

Ser Leu Phe Pro Asp Ile Asp Gln Arg Lys Ser Ser Ile Ser Gln Met
    50                  55                  60

Tyr Pro Phe Leu Ser Lys His Ile Gly Ser Lys Cys Arg His Arg Gly
65                  70                  75                  80

Phe Thr His Ser Leu Leu Cys Leu Ser Leu Ile Val Leu Thr Leu Tyr
                85                  90                  95

Ile Ile Leu Asn Val Ser Asn Phe Asn Ile Ile Leu Leu Ile Ile Ser
            100                 105                 110

Ile Gly Phe Ile Ala Gly Tyr Ile Ser His Leu Val Leu Asp Phe Leu
        115                 120                 125

Thr Ser Glu Gly Ile Glu Leu Phe Tyr Pro Trp Lys Thr Asn Phe Lys
    130                 135                 140

Ile Ala Lys Ile Lys Thr Gly Ser Lys Thr Glu Lys Ile Ile Asn Lys
145                 150                 155                 160

Ile Leu Lys Ile Phe Asn Phe Leu Leu Ile Ile Tyr Asn Ile Val Leu
                165                 170                 175

Ile Leu Ser Asp Ile Leu Gly Ile Asn Ile Leu Ser Phe Ile Ser Lys
            180                 185                 190

Ser Thr Leu Phe
        195

<210>  16
<211>  5628
<212>  DNA
<213>  Clostridium perfringens

<220>
<221>  CDS
<222>  (673)..(5628)
<223>  TpeL gene

<400>  16
gatcttaatg aacctaatac agatatattt gtttatgatt taatagacaa gctttcagaa      60

aaagaaagaa aaataattaa atataaatac atacatggaa aaagtgatgt tgaaataggg     120

aaattactta attattctag acagcacgta aaccaaataa aaaatagagc attaaaaaat     180

ttgaagaaat ttttagagga gtgatttaat ctaaatatta aaattaaata tttataggag     240

gttaattgtg gattcagaac tgtttaaaat tatggctact caaggagctt ttgctttact     300

attttcctac ttgttatttt atgtattaaa agaaaatagc aaaagagaag aaaattatca     360

aaatataatt aaggagctta cagaattact accaattata aaaagtgatg ttgaagacat     420

aaaaaataaa ttgaataata attagaaaaa atttataaaa aatataaatt ttaaattttt 480

atttacaaaa tttaaaatta gaactcttta tataatgtat aagaaattta attctaagta 540

tataaaatac taaaggaggg ttaagaagaa taaaatttga aaggaaaaaa tttgtgcaaa 600

ttcaaattta taattagatt gaagggagga tttgatgtaa atataaatta agttttacat 660

caacaataat at atg ggg tta atg tca aaa gaa caa tta att ata tta gca 711
Met Gly Leu Met Ser Lys Glu Gln Leu Ile Ile Leu Ala
1               5                   10

aaa aat tca agt cca aaa gaa gga gaa tat aaa aaa att tta gaa tta 759
Lys Asn Ser Ser Pro Lys Glu Gly Glu Tyr Lys Lys Ile Leu Glu Leu
    15                  20                  25

cta gat gaa tat aat tta tta aat aat tct gtt gaa aaa aat agt ata 807
Leu Asp Glu Tyr Asn Leu Leu Asn Asn Ser Val Glu Lys Asn Ser Ile
30                  35                  40                  45

gat tta tat tta aaa ctt aat gaa tta tct aaa tca att gat att tat 855
Asp Leu Tyr Leu Lys Leu Asn Glu Leu Ser Lys Ser Ile Asp Ile Tyr
                50                  55                  60

tta aaa aaa tat aag aat tca aag aga aat aat gca tta tat caa tta 903
Leu Lys Lys Tyr Lys Asn Ser Lys Arg Asn Asn Ala Leu Tyr Gln Leu
            65                  70                  75

aaa tct gat tta aca aag gaa gtt att gaa ata aaa gat act aat tta 951
Lys Ser Asp Leu Thr Lys Glu Val Ile Glu Ile Lys Asp Thr Asn Leu
        80                  85                  90

aaa cct ttg gaa aaa aat att cat ttt gtt tgg gtt gga gga atg ata 999
Lys Pro Leu Glu Lys Asn Ile His Phe Val Trp Val Gly Gly Met Ile
    95                  100                 105

aat aat ata tct att gat tat ata aat caa tgg aaa gat att aat agc 1047
Asn Asn Ile Ser Ile Asp Tyr Ile Asn Gln Trp Lys Asp Ile Asn Ser
110                 115                 120                 125

gat tat gaa act att ata tgg tat gat agt gaa gct ttg tta gta aat 1095
Asp Tyr Glu Thr Ile Ile Trp Tyr Asp Ser Glu Ala Leu Leu Val Asn
                130                 135                 140

ata tta aaa aaa gct ata ata gac tca tca aat aaa gaa gtt tta act 1143
Ile Leu Lys Lys Ala Ile Ile Asp Ser Ser Asn Lys Glu Val Leu Thr
            145                 150                 155

aaa tat gaa tca gta tta aat gat aat agc ttt gat tca aat aaa ttt 1191
Lys Tyr Glu Ser Val Leu Asn Asp Asn Ser Phe Asp Ser Asn Lys Phe
        160                 165                 170

tat aga gaa aga atg gaa gtt att ttt aga aag caa aaa gag ttt aat 1239
Tyr Arg Glu Arg Met Glu Val Ile Phe Arg Lys Gln Lys Glu Phe Asn
    175                 180                 185

aat tat tat aat aca aat gat aac tat act aaa tct tta aat gat gta 1287
Asn Tyr Tyr Asn Thr Asn Asp Asn Tyr Thr Lys Ser Leu Asn Asp Val
190                 195                 200                 205

att aaa gtt tat tta ata gaa aaa tat tta aaa act gat gaa gaa tta 1335
Ile Lys Val Tyr Leu Ile Glu Lys Tyr Leu Lys Thr Asp Glu Glu Leu
                210                 215                 220

gaa aaa tat att aat gaa agt aaa gaa gtt ttt aag gct aat gga gct          1383
Glu Lys Tyr Ile Asn Glu Ser Lys Glu Val Phe Lys Ala Asn Gly Ala
        225                 230                 235

aaa gat ata aga gaa tac gat ata cta gat gat gtt gaa tta aaa tca          1431
Lys Asp Ile Arg Glu Tyr Asp Ile Leu Asp Asp Val Glu Leu Lys Ser
    240                 245                 250

ata tat gag caa gag tta tta atg aga ttt aat tta gca tct gca tca          1479
Ile Tyr Glu Gln Glu Leu Leu Met Arg Phe Asn Leu Ala Ser Ala Ser
    255                 260                 265

gat att att aga gtg att gta tta aat aaa tta ggt ggg att tat tta          1527
Asp Ile Ile Arg Val Ile Val Leu Asn Lys Leu Gly Gly Ile Tyr Leu
270                 275                 280                 285

gat gta gat gtg ctt cct gga ata aaa aaa cat att ttt aaa gat att          1575
Asp Val Asp Val Leu Pro Gly Ile Lys Lys His Ile Phe Lys Asp Ile
                290                 295                 300

aat aaa cct aca aat att tct gaa aat aaa tgg caa atg att caa tta          1623
Asn Lys Pro Thr Asn Ile Ser Glu Asn Lys Trp Gln Met Ile Gln Leu
        305                 310                 315

gaa act att atg aaa tat aaa caa tat ata aaa gga tat aca gaa aat          1671
Glu Thr Ile Met Lys Tyr Lys Gln Tyr Ile Lys Gly Tyr Thr Glu Asn
        320                 325                 330

tca ttt aaa aat tta cca agt gat ttg cag gaa atg tta caa gaa aaa          1719
Ser Phe Lys Asn Leu Pro Ser Asp Leu Gln Glu Met Leu Gln Glu Lys
    335                 340                 345

gtt gtt gaa aaa aat ctt aaa agt gat ata ttt cag aga tta ggg gat          1767
Val Val Glu Lys Asn Leu Lys Ser Asp Ile Phe Gln Arg Leu Gly Asp
350                 355                 360                 365

ata ttt att tca gaa tta gat aca aaa ata gct ttt atg ttt ggg aaa          1815
Ile Phe Ile Ser Glu Leu Asp Thr Lys Ile Ala Phe Met Phe Gly Lys
                370                 375                 380

att gct aat caa gta tta ata tct aaa aaa aat tca tac tca tta aat          1863
Ile Ala Asn Gln Val Leu Ile Ser Lys Lys Asn Ser Tyr Ser Leu Asn
        385                 390                 395

tta ata att aat caa atc aag aat aga tat aat att att aat aaa tgt          1911
Leu Ile Ile Asn Gln Ile Lys Asn Arg Tyr Asn Ile Ile Asn Lys Cys
        400                 405                 410

tta tct tcg gct att gaa aaa gga tca aat ttt aat aat aca gtt gat          1959
Leu Ser Ser Ala Ile Glu Lys Gly Ser Asn Phe Asn Asn Thr Val Asp
    415                 420                 425

ata ttc att caa caa tta aat gaa ttc tat gtt aat gaa ggc ttt ttt          2007
Ile Phe Ile Gln Gln Leu Asn Glu Phe Tyr Val Asn Glu Gly Phe Phe
430                 435                 440                 445

gtt agt aaa gta atg gga tat tta ggt gat gga tat atg cct gat atg          2055
Val Ser Lys Val Met Gly Tyr Leu Gly Asp Gly Tyr Met Pro Asp Met
                450                 455                 460

aga gcg aca ctt aat ata agt ggt cca gga ata tat aca gct gct tat          2103
Arg Ala Thr Leu Asn Ile Ser Gly Pro Gly Ile Tyr Thr Ala Ala Tyr

            465                 470                 475

tat gat tta tta tat ttt aat gag agg tca tta aat cct caa att tta 2151
Tyr Asp Leu Leu Tyr Phe Asn Glu Arg Ser Leu Asn Pro Gln Ile Leu
        480                 485                 490

caa gaa gat ttg aaa tat ttt gaa gtt cca caa gct cta ata tca caa 2199
Gln Glu Asp Leu Lys Tyr Phe Glu Val Pro Gln Ala Leu Ile Ser Gln
    495                 500                 505

caa aca gaa caa gaa ata aat agt tca tgg aca ttt aat caa gtt aaa 2247
Gln Thr Glu Gln Glu Ile Asn Ser Ser Trp Thr Phe Asn Gln Val Lys
510                 515                 520                 525

tca caa ata gaa tat aaa aag cta gta gaa aaa tat act aat aaa tca 2295
Ser Gln Ile Glu Tyr Lys Lys Leu Val Glu Lys Tyr Thr Asn Lys Ser
                530                 535                 540

tta agt gaa aat gat aaa cta aat ttt aat gag aat aaa atc att gat 2343
Leu Ser Glu Asn Asp Lys Leu Asn Phe Asn Glu Asn Lys Ile Ile Asp
            545                 550                 555

aaa gta gaa tta cta aat cga att aat agt aat aat tta att aat ttt 2391
Lys Val Glu Leu Leu Asn Arg Ile Asn Ser Asn Asn Leu Ile Asn Phe
        560                 565                 570

gat gat aaa gaa tac tta aga tat ata att caa tta caa ggt gat aaa 2439
Asp Asp Lys Glu Tyr Leu Arg Tyr Ile Ile Gln Leu Gln Gly Asp Lys
    575                 580                 585

gta agt tat gag gct gca ata aat tta ttt att aaa aat cct tca aat 2487
Val Ser Tyr Glu Ala Ala Ile Asn Leu Phe Ile Lys Asn Pro Ser Asn
590                 595                 600                 605

tca ata cta gtt caa gaa att aat aat ata agc tac tat ttt aat tca 2535
Ser Ile Leu Val Gln Glu Ile Asn Asn Ile Ser Tyr Tyr Phe Asn Ser
                610                 615                 620

gaa tat aaa tca ata gat tct ata caa ttt gat aat att cct gaa ata 2583
Glu Tyr Lys Ser Ile Asp Ser Ile Gln Phe Asp Asn Ile Pro Glu Ile
            625                 630                 635

tta aaa ggt aaa aat aaa att aaa tta aca ttt ata ggt cat gga gaa 2631
Leu Lys Gly Lys Asn Lys Ile Lys Leu Thr Phe Ile Gly His Gly Glu
        640                 645                 650

gaa gaa ttt aat aca gag aga ttt gct tca tta aca gta aag gag ttt 2679
Glu Glu Phe Asn Thr Glu Arg Phe Ala Ser Leu Thr Val Lys Glu Phe
    655                 660                 665

tca aaa aaa ata tat aaa gtt tta gat atg ata aag agc aat acg aat 2727
Ser Lys Lys Ile Tyr Lys Val Leu Asp Met Ile Lys Ser Asn Thr Asn
670                 675                 680                 685

gtt aaa gaa att caa ata gat ttg tta gga tgt aat atg ttt tca tat 2775
Val Lys Glu Ile Gln Ile Asp Leu Leu Gly Cys Asn Met Phe Ser Tyr
                690                 695                 700

aat ata aat gta gaa gaa aca tat cct ggg aaa tta tta aaa gta gta 2823
Asn Ile Asn Val Glu Glu Thr Tyr Pro Gly Lys Leu Leu Lys Val Val
            705                 710                 715

tta gat tat gta gat aaa att tat aat gca gat att aaa cca gaa ata 2871

Leu Asp Tyr Val Asp Lys Ile Tyr Asn Ala Asp Ile Lys Pro Glu Ile
        720                 725                 730

aaa ata agt gcc aat caa tat gaa gtc aga ata aat aaa gat ggg aaa        2919
Lys Ile Ser Ala Asn Gln Tyr Glu Val Arg Ile Asn Lys Asp Gly Lys
    735                 740                 745

aaa gaa tta cta tct cat tca gga gag tgg ctt tct aaa gaa gaa gct        2967
Lys Glu Leu Leu Ser His Ser Gly Glu Trp Leu Ser Lys Glu Glu Ala
750                 755                 760                 765

ata ata aaa gat att gcg agt aaa gaa att att tat ttt gat aca aga        3015
Ile Ile Lys Asp Ile Ala Ser Lys Glu Ile Ile Tyr Phe Asp Thr Arg
                770                 775                 780

gaa aat act att aaa gca gaa gct aaa aat att atg gag ttg ata act        3063
Glu Asn Thr Ile Lys Ala Glu Ala Lys Asn Ile Met Glu Leu Ile Thr
            785                 790                 795

ttt aga aat agt cta gat aaa aaa tta aat gat tta gaa agt tta att        3111
Phe Arg Asn Ser Leu Asp Lys Lys Leu Asn Asp Leu Glu Ser Leu Ile
        800                 805                 810

aat aat aat ttt att gta aat gaa gta tat gaa caa tta aca tca aat        3159
Asn Asn Asn Phe Ile Val Asn Glu Val Tyr Glu Gln Leu Thr Ser Asn
    815                 820                 825

aat tta tat aga aca agt ttt aga aat tct ttt tta gat act ata aat        3207
Asn Leu Tyr Arg Thr Ser Phe Arg Asn Ser Phe Leu Asp Thr Ile Asn
830                 835                 840                 845

ttt ata gaa gat gtt tct caa gag tta tat gaa ata aag ata aaa aat        3255
Phe Ile Glu Asp Val Ser Gln Glu Leu Tyr Glu Ile Lys Ile Lys Asn
                850                 855                 860

aat ata aac gat gat tat atc atg gca tta gat gaa ata aag caa gat        3303
Asn Ile Asn Asp Asp Tyr Ile Met Ala Leu Asp Glu Ile Lys Gln Asp
            865                 870                 875

aat aat att tca gaa att aaa ttt att aat att aat tca gga gaa ttt        3351
Asn Asn Ile Ser Glu Ile Lys Phe Ile Asn Ile Asn Ser Gly Glu Phe
        880                 885                 890

agg gtt gta aga gaa aat agt agg tta att aat aaa ttt aaa gaa gat        3399
Arg Val Val Arg Glu Asn Ser Arg Leu Ile Asn Lys Phe Lys Glu Asp
    895                 900                 905

ttt aaa ttt atg att aat aat att aag agg ttg ata aaa ttt agt gat        3447
Phe Lys Phe Met Ile Asn Asn Ile Lys Arg Leu Ile Lys Phe Ser Asp
910                 915                 920                 925

aat aac aag cta ata aca tca ttt gaa tta aag aat ata gag tca agc        3495
Asn Asn Lys Leu Ile Thr Ser Phe Glu Leu Lys Asn Ile Glu Ser Ser
                930                 935                 940

agt gga gta agt aca tta aat aca agt ttt tta att caa tct atg att        3543
Ser Gly Val Ser Thr Leu Asn Thr Ser Phe Leu Ile Gln Ser Met Ile
            945                 950                 955

gat tat aaa gcg caa aac ttt gac ttc aat aaa tta agt aca tca gta        3591
Asp Tyr Lys Ala Gln Asn Phe Asp Phe Asn Lys Leu Ser Thr Ser Val
        960                 965                 970

aaa gta cag att tat tgt caa ata act aat ata agt tta tca gaa att 3639
Lys Val Gln Ile Tyr Cys Gln Ile Thr Asn Ile Ser Leu Ser Glu Ile
    975                 980                 985

caa gat gct agt aat tta gta aaa att ata gct gaa gca aat gaa att 3687
Gln Asp Ala Ser Asn Leu Val Lys Ile Ile Ala Glu Ala Asn Glu Ile
990                 995                 1000                1005

gaa ata aat tta ata cca aca tta gca aat gct ata cca tta ata 3732
Glu Ile Asn Leu Ile Pro Thr Leu Ala Asn Ala Ile Pro Leu Ile
                1010                1015                1020

aca aca ata gtt gat gga ata aat ttg att gca aat ata gat gaa 3777
Thr Thr Ile Val Asp Gly Ile Asn Leu Ile Ala Asn Ile Asp Glu
                1025                1030                1035

tta ata aat act aaa gat gaa tta cta aga aag gaa tta gca gct 3822
Leu Ile Asn Thr Lys Asp Glu Leu Leu Arg Lys Glu Leu Ala Ala
                1040                1045                1050

aga att gga att att tca tct aat atg aca gca gct att agt tct 3867
Arg Ile Gly Ile Ile Ser Ser Asn Met Thr Ala Ala Ile Ser Ser
                1055                1060                1065

tat att tta tat ttt act gaa ttt gga gaa gta ttt aat cct tta 3912
Tyr Ile Leu Tyr Phe Thr Glu Phe Gly Glu Val Phe Asn Pro Leu
                1070                1075                1080

tta gtg cca att gca ggt ata tca agt ggt att cct act tta gta 3957
Leu Val Pro Ile Ala Gly Ile Ser Ser Gly Ile Pro Thr Leu Val
                1085                1090                1095

aat aat att tta att tta gag gaa aaa tct aaa gaa ata aca gaa 4002
Asn Asn Ile Leu Ile Leu Glu Glu Lys Ser Lys Glu Ile Thr Glu
                1100                1105                1110

tat ttt tca cat ata agt aaa gta gaa agt gat gga tta ttt aaa 4047
Tyr Phe Ser His Ile Ser Lys Val Glu Ser Asp Gly Leu Phe Lys
                1115                1120                1125

gtg tct gat aat aac agt ata ttg att cca ctt gat gat att gca 4092
Val Ser Asp Asn Asn Ser Ile Leu Ile Pro Leu Asp Asp Ile Ala
                1130                1135                1140

att tct aag att gat ttt aat aaa aga gaa gtt atc tta gat aaa 4137
Ile Ser Lys Ile Asp Phe Asn Lys Arg Glu Val Ile Leu Asp Lys
                1145                1150                1155

tta gat ata tgg gca atg gaa gga gga tca gga tta agt ggt aaa 4182
Leu Asp Ile Trp Ala Met Glu Gly Gly Ser Gly Leu Ser Gly Lys
                1160                1165                1170

gaa act ttt ttt tct gct ccg tat ata aat gaa aat ctt cct aaa 4227
Glu Thr Phe Phe Ser Ala Pro Tyr Ile Asn Glu Asn Leu Pro Lys
                1175                1180                1185

tta tct att gat aat ttg ctt aag ata gat ata aat aaa ata gat 4272
Leu Ser Ile Asp Asn Leu Leu Lys Ile Asp Ile Asn Lys Ile Asp
                1190                1195                1200

ttt tca att aaa ggt atg atg cta cct aat ggc gtt agt aaa aca 4317
Phe Ser Ile Lys Gly Met Met Leu Pro Asn Gly Val Ser Lys Thr
                1205                1210                1215

tta ggt tat gag ttc aat aca gtt gcc aat ata tat gaa tta gaa 4362
Leu Gly Tyr Glu Phe Asn Thr Val Ala Asn Ile Tyr Glu Leu Glu
                1220                1225                1230

aat gat ggt gta aat tta tta aat aga att aga gat aat tat cca 4407
Asn Asp Gly Val Asn Leu Leu Asn Arg Ile Arg Asp Asn Tyr Pro
                1235                1240                1245

gga cag ttt tat tgg aga tat tat gct act cta ttt aat tat ggt 4452
Gly Gln Phe Tyr Trp Arg Tyr Tyr Ala Thr Leu Phe Asn Tyr Gly
                1250                1255                1260

atg gta aat tta aaa att aat tat cat gac aca gat gta aaa ata 4497
Met Val Asn Leu Lys Ile Asn Tyr His Asp Thr Asp Val Lys Ile
                1265                1270                1275

aat tta gat aat gtt gat cgt atg ttt ata gta ccg aca tta aca 4542
Asn Leu Asp Asn Val Asp Arg Met Phe Ile Val Pro Thr Leu Thr
                1280                1285                1290

ata gat caa gct aga gaa aaa ctt tca tat aat ttt aat gga gca 4587
Ile Asp Gln Ala Arg Glu Lys Leu Ser Tyr Asn Phe Asn Gly Ala
                1295                1300                1305

gga gct aat tat tat ata tac ctt tcg tct caa cca ata aaa ata 4632
Gly Ala Asn Tyr Tyr Ile Tyr Leu Ser Ser Gln Pro Ile Lys Ile
                1310                1315                1320

ttt att aat ggg act aaa gaa gat aat tgg att ctt aat ata gat 4677
Phe Ile Asn Gly Thr Lys Glu Asp Asn Trp Ile Leu Asn Ile Asp
                1325                1330                1335

gat att gtt aaa gaa gtt aca att gtt aat aat agt ata gta aga 4722
Asp Ile Val Lys Glu Val Thr Ile Val Asn Asn Ser Ile Val Arg
                1340                1345                1350

gga aag ttt att gaa aat ata ttt aaa aaa tta act att gag gat 4767
Gly Lys Phe Ile Glu Asn Ile Phe Lys Lys Leu Thr Ile Glu Asp
                1355                1360                1365

aat aaa ata att ata gga aat caa aaa ata tat tta aaa aat aac 4812
Asn Lys Ile Ile Ile Gly Asn Gln Lys Ile Tyr Leu Lys Asn Asn
                1370                1375                1380

aaa aca aat ata cat ttt tct gta tct att tta gat gga gtg aat 4857
Lys Thr Asn Ile His Phe Ser Val Ser Ile Leu Asp Gly Val Asn
                1385                1390                1395

tta atg gta gaa gta gac ttt aat aat aaa atg tat cat tta tca 4902
Leu Met Val Glu Val Asp Phe Asn Asn Lys Met Tyr His Leu Ser
                1400                1405                1410

ctt caa gga aac gaa aaa aca ata tgt aat aat atg aat att ata 4947
Leu Gln Gly Asn Glu Lys Thr Ile Cys Asn Asn Met Asn Ile Ile
                1415                1420                1425

caa aat aac ata aat aaa tta ttt ggc tct tca aat ata aaa tca 4992
Gln Asn Asn Ile Asn Lys Leu Phe Gly Ser Ser Asn Ile Lys Ser
                1430                1435                1440

ata cca tat ttc tat aaa aat aat gga tta gag aat tgt att gga 5037
Ile Pro Tyr Phe Tyr Lys Asn Asn Gly Leu Glu Asn Cys Ile Gly

1445                1450                1455

gta ttc tca ata aaa  aac aat gaa ttt att  tat atg tta aaa tat        5082
Val Phe Ser Ile Lys  Asn Asn Glu Phe Ile  Tyr Met Leu Lys Tyr
                1460                1465                1470

gat gat aaa aat aaa  ata tat aaa tat aaa  gat aat aat tta gta        5127
Asp Asp Lys Asn Lys  Ile Tyr Lys Tyr Lys  Asp Asn Asn Leu Val
                1475                1480                1485

tgt ggt ttt aat tta  ttt gat ata tct tat  gta gat ata att tct        5172
Cys Gly Phe Asn Leu  Phe Asp Ile Ser Tyr  Val Asp Ile Ile Ser
                1490                1495                1500

aat gga gat aag aat  tat ttg aaa gga att  tgg aat aca tca gtt        5217
Asn Gly Asp Lys Asn  Tyr Leu Lys Gly Ile  Trp Asn Thr Ser Val
                1505                1510                1515

aat aat tca gaa tat  aaa att gaa ttt tta  gtt gaa tta ata gac        5262
Asn Asn Ser Glu Tyr  Lys Ile Glu Phe Leu  Val Glu Leu Ile Asp
                1520                1525                1530

caa aat act att gaa  atc att aaa ttg aat  tta agt gat gga tta        5307
Gln Asn Thr Ile Glu  Ile Ile Lys Leu Asn  Leu Ser Asp Gly Leu
                1535                1540                1545

ata aaa tca ttt tta  act ata tta gat aat  tta gaa agt tta gat        5352
Ile Lys Ser Phe Leu  Thr Ile Leu Asp Asn  Leu Glu Ser Leu Asp
                1550                1555                1560

gta aaa att tca aca  ata tat gat ttt tta  aaa aat att ctt ttt        5397
Val Lys Ile Ser Thr  Ile Tyr Asp Phe Leu  Lys Asn Ile Leu Phe
                1565                1570                1575

agg gat gta ttt tta  gaa aat gag tca tta  aat ttt att gta agt        5442
Arg Asp Val Phe Leu  Glu Asn Glu Ser Leu  Asn Phe Ile Val Ser
                1580                1585                1590

aaa ggc ttt gtt ttg  aca ggg aat aat tca  aaa aat aaa tac gag        5487
Lys Gly Phe Val Leu  Thr Gly Asn Asn Ser  Lys Asn Lys Tyr Glu
                1595                1600                1605

ttt ata tgt aat agt  gaa aat ata caa tta  tat att act gaa ctt        5532
Phe Ile Cys Asn Ser  Glu Asn Ile Gln Leu  Tyr Ile Thr Glu Leu
                1610                1615                1620

tac act aat ggt act  aaa ttt agg aat tgg  ctt gac aat ggt aag        5577
Tyr Thr Asn Gly Thr  Lys Phe Arg Asn Trp  Leu Asp Asn Gly Lys
                1625                1630                1635

ata tta gta tca tca  aca ggt gaa agt tca  aat aat tta att gac        5622
Ile Leu Val Ser Ser  Thr Gly Glu Ser Ser  Asn Asn Leu Ile Asp
                1640                1645                1650

ata taa                                                              5628
Ile

<210>  17
<211>  1651
<212>  PRT
<213>  Clostridium perfringens

<400>  17

Met Gly Leu Met Ser Lys Glu Gln Leu Ile Ile Leu Ala Lys Asn Ser
1               5                   10                  15

Ser Pro Lys Glu Gly Glu Tyr Lys Lys Ile Leu Glu Leu Leu Asp Glu
            20                  25                  30

Tyr Asn Leu Leu Asn Asn Ser Val Glu Lys Asn Ser Ile Asp Leu Tyr
        35                  40                  45

Leu Lys Leu Asn Glu Leu Ser Lys Ser Ile Asp Ile Tyr Leu Lys Lys
    50                  55                  60

Tyr Lys Asn Ser Lys Arg Asn Asn Ala Leu Tyr Gln Leu Lys Ser Asp
65                  70                  75                  80

Leu Thr Lys Glu Val Ile Glu Ile Lys Asp Thr Asn Leu Lys Pro Leu
                85                  90                  95

Glu Lys Asn Ile His Phe Val Trp Val Gly Gly Met Ile Asn Asn Ile
            100                 105                 110

Ser Ile Asp Tyr Ile Asn Gln Trp Lys Asp Ile Asn Ser Asp Tyr Glu
        115                 120                 125

Thr Ile Ile Trp Tyr Asp Ser Glu Ala Leu Leu Val Asn Ile Leu Lys
    130                 135                 140

Lys Ala Ile Ile Asp Ser Ser Asn Lys Glu Val Leu Thr Lys Tyr Glu
145                 150                 155                 160

Ser Val Leu Asn Asp Asn Ser Phe Asp Ser Asn Lys Phe Tyr Arg Glu
                165                 170                 175

Arg Met Glu Val Ile Phe Arg Lys Gln Lys Glu Phe Asn Asn Tyr Tyr
            180                 185                 190

Asn Thr Asn Asp Asn Tyr Thr Lys Ser Leu Asn Asp Val Ile Lys Val
        195                 200                 205

Tyr Leu Ile Glu Lys Tyr Leu Lys Thr Asp Glu Glu Leu Glu Lys Tyr
    210                 215                 220

Ile Asn Glu Ser Lys Glu Val Phe Lys Ala Asn Gly Ala Lys Asp Ile
225                 230                 235                 240

Arg Glu Tyr Asp Ile Leu Asp Asp Val Glu Leu Lys Ser Ile Tyr Glu
                245                 250                 255

Gln Glu Leu Leu Met Arg Phe Asn Leu Ala Ser Ala Ser Asp Ile Ile
            260                 265                 270

Arg Val Ile Val Leu Asn Lys Leu Gly Gly Ile Tyr Leu Asp Val Asp
        275                 280                 285

Val Leu Pro Gly Ile Lys Lys His Ile Phe Lys Asp Ile Asn Lys Pro
    290                 295                 300

Thr Asn Ile Ser Glu Asn Lys Trp Gln Met Ile Gln Leu Glu Thr Ile
305                 310                 315                 320

Met Lys Tyr Lys Gln Tyr Ile Lys Gly Tyr Thr Glu Asn Ser Phe Lys
                325                 330                 335

Asn Leu Pro Ser Asp Leu Gln Glu Met Leu Gln Glu Lys Val Val Glu
            340                 345                 350

Lys Asn Leu Lys Ser Asp Ile Phe Gln Arg Leu Gly Asp Ile Phe Ile
        355                 360                 365

Ser Glu Leu Asp Thr Lys Ile Ala Phe Met Phe Gly Lys Ile Ala Asn
    370                 375                 380

Gln Val Leu Ile Ser Lys Lys Asn Ser Tyr Ser Leu Asn Leu Ile Ile
385                 390                 395                 400

Asn Gln Ile Lys Asn Arg Tyr Asn Ile Ile Asn Lys Cys Leu Ser Ser
                405                 410                 415

Ala Ile Glu Lys Gly Ser Asn Phe Asn Asn Thr Val Asp Ile Phe Ile
            420                 425                 430

Gln Gln Leu Asn Glu Phe Tyr Val Asn Glu Gly Phe Phe Val Ser Lys
        435                 440                 445

Val Met Gly Tyr Leu Gly Asp Gly Tyr Met Pro Asp Met Arg Ala Thr
    450                 455                 460

Leu Asn Ile Ser Gly Pro Gly Ile Tyr Thr Ala Ala Tyr Tyr Asp Leu
465                 470                 475                 480

Leu Tyr Phe Asn Glu Arg Ser Leu Asn Pro Gln Ile Leu Gln Glu Asp
                485                 490                 495

Leu Lys Tyr Phe Glu Val Pro Gln Ala Leu Ile Ser Gln Gln Thr Glu
            500                 505                 510

Gln Glu Ile Asn Ser Ser Trp Thr Phe Asn Gln Val Lys Ser Gln Ile
        515                 520                 525

Glu Tyr Lys Lys Leu Val Glu Lys Tyr Thr Asn Lys Ser Leu Ser Glu
    530                 535                 540

Asn Asp Lys Leu Asn Phe Asn Glu Asn Lys Ile Ile Asp Lys Val Glu
545                 550                 555                 560

Leu Leu Asn Arg Ile Asn Ser Asn Asn Leu Ile Asn Phe Asp Asp Lys
                565                 570                 575

Glu Tyr Leu Arg Tyr Ile Ile Gln Leu Gln Gly Asp Lys Val Ser Tyr
            580                 585                 590

Glu Ala Ala Ile Asn Leu Phe Ile Lys Asn Pro Ser Asn Ser Ile Leu
        595                 600                 605

Val Gln Glu Ile Asn Asn Ile Ser Tyr Tyr Phe Asn Ser Glu Tyr Lys
    610                 615                 620

Ser Ile Asp Ser Ile Gln Phe Asp Asn Ile Pro Glu Ile Leu Lys Gly
625                 630                 635                 640

Lys Asn Lys Ile Lys Leu Thr Phe Ile Gly His Gly Glu Glu Glu Phe
                645                 650                 655

Asn Thr Glu Arg Phe Ala Ser Leu Thr Val Lys Glu Phe Ser Lys Lys
            660                 665                 670

Ile Tyr Lys Val Leu Asp Met Ile Lys Ser Asn Thr Asn Val Lys Glu
        675                 680                 685

Ile Gln Ile Asp Leu Leu Gly Cys Asn Met Phe Ser Tyr Asn Ile Asn
    690                 695                 700

Val Glu Glu Thr Tyr Pro Gly Lys Leu Leu Lys Val Val Leu Asp Tyr
705                 710                 715                 720

Val Asp Lys Ile Tyr Asn Ala Asp Ile Lys Pro Glu Ile Lys Ile Ser
                725                 730                 735

Ala Asn Gln Tyr Glu Val Arg Ile Asn Lys Asp Gly Lys Lys Glu Leu
            740                 745                 750

Leu Ser His Ser Gly Glu Trp Leu Ser Lys Glu Glu Ala Ile Ile Lys
        755                 760                 765

Asp Ile Ala Ser Lys Glu Ile Ile Tyr Phe Asp Thr Arg Glu Asn Thr
    770                 775                 780

Ile Lys Ala Glu Ala Lys Asn Ile Met Glu Leu Ile Thr Phe Arg Asn
785                 790                 795                 800

Ser Leu Asp Lys Lys Leu Asn Asp Leu Glu Ser Leu Ile Asn Asn Asn
                805                 810                 815

Phe Ile Val Asn Glu Val Tyr Glu Gln Leu Thr Ser Asn Asn Leu Tyr
            820                 825                 830

Arg Thr Ser Phe Arg Asn Ser Phe Leu Asp Thr Ile Asn Phe Ile Glu
        835                 840                 845

Asp Val Ser Gln Glu Leu Tyr Glu Ile Lys Ile Lys Asn Asn Ile Asn
    850                 855                 860

Asp Asp Tyr Ile Met Ala Leu Asp Glu Ile Lys Gln Asp Asn Asn Ile
865                 870                 875                 880

Ser Glu Ile Lys Phe Ile Asn Ile Asn Ser Gly Glu Phe Arg Val Val
                885                 890                 895

Arg Glu Asn Ser Arg Leu Ile Asn Lys Phe Lys Glu Asp Phe Lys Phe
            900                 905                 910

Met Ile Asn Asn Ile Lys Arg Leu Ile Lys Phe Ser Asp Asn Asn Lys
        915                 920                 925

Leu Ile Thr Ser Phe Glu Leu Lys Asn Ile Glu Ser Ser Ser Gly Val
    930                 935                 940

Ser Thr Leu Asn Thr Ser Phe Leu Ile Gln Ser Met Ile Asp Tyr Lys
945                 950                 955                 960

Ala Gln Asn Phe Asp Phe Asn Lys Leu Ser Thr Ser Val Lys Val Gln
                965                 970                 975

Ile Tyr Cys Gln Ile Thr Asn Ile Ser Leu Ser Glu Ile Gln Asp Ala
            980                 985                 990

Ser Asn Leu Val Lys Ile Ile Ala  Glu Ala Asn Glu Ile  Glu Ile Asn

        995                 1000                 1005

Leu Ile Pro Thr Leu Ala Asn Ala Ile Pro Leu Ile Thr Thr Ile
    1010                1015                1020

Val Asp Gly Ile Asn Leu Ile Ala Asn Ile Asp Glu Leu Ile Asn
    1025                1030                1035

Thr Lys Asp Glu Leu Leu Arg Lys Glu Leu Ala Ala Arg Ile Gly
    1040                1045                1050

Ile Ile Ser Ser Asn Met Thr Ala Ala Ile Ser Ser Tyr Ile Leu
    1055                1060                1065

Tyr Phe Thr Glu Phe Gly Glu Val Phe Asn Pro Leu Leu Val Pro
    1070                1075                1080

Ile Ala Gly Ile Ser Ser Gly Ile Pro Thr Leu Val Asn Asn Ile
    1085                1090                1095

Leu Ile Leu Glu Glu Lys Ser Lys Glu Ile Thr Glu Tyr Phe Ser
    1100                1105                1110

His Ile Ser Lys Val Glu Ser Asp Gly Leu Phe Lys Val Ser Asp
    1115                1120                1125

Asn Asn Ser Ile Leu Ile Pro Leu Asp Asp Ile Ala Ile Ser Lys
    1130                1135                1140

Ile Asp Phe Asn Lys Arg Glu Val Ile Leu Asp Lys Leu Asp Ile
    1145                1150                1155

Trp Ala Met Glu Gly Gly Ser Gly Leu Ser Gly Lys Glu Thr Phe
    1160                1165                1170

Phe Ser Ala Pro Tyr Ile Asn Glu Asn Leu Pro Lys Leu Ser Ile
    1175                1180                1185

Asp Asn Leu Leu Lys Ile Asp Ile Asn Lys Ile Asp Phe Ser Ile
    1190                1195                1200

Lys Gly Met Met Leu Pro Asn Gly Val Ser Lys Thr Leu Gly Tyr
    1205                1210                1215

Glu Phe Asn Thr Val Ala Asn Ile Tyr Glu Leu Glu Asn Asp Gly
    1220                1225                1230

Val Asn Leu Leu Asn Arg Ile Arg Asp Asn Tyr Pro Gly Gln Phe
    1235                1240                1245

Tyr Trp Arg Tyr Tyr Ala Thr Leu Phe Asn Tyr Gly Met Val Asn
    1250                1255                1260

Leu Lys Ile Asn Tyr His Asp Thr Asp Val Lys Ile Asn Leu Asp
    1265                1270                1275

Asn Val Asp Arg Met Phe Ile Val Pro Thr Leu Thr Ile Asp Gln
    1280                1285                1290

Ala Arg Glu Lys Leu Ser Tyr Asn Phe Asn Gly Ala Gly Ala Asn
    1295                1300                1305

Tyr Tyr Ile Tyr Leu Ser Ser Gln Pro Ile Lys Ile Phe Ile Asn
    1310                1315                1320

Gly Thr Lys Glu Asp Asn Trp Ile Leu Asn Ile Asp Asp Ile Val
    1325                1330                1335

Lys Glu Val Thr Ile Val Asn Asn Ser Ile Val Arg Gly Lys Phe
    1340                1345                1350

Ile Glu Asn Ile Phe Lys Lys Leu Thr Ile Glu Asp Asn Lys Ile
    1355                1360                1365

Ile Ile Gly Asn Gln Lys Ile Tyr Leu Lys Asn Asn Lys Thr Asn
    1370                1375                1380

Ile His Phe Ser Val Ser Ile Leu Asp Gly Val Asn Leu Met Val
    1385                1390                1395

Glu Val Asp Phe Asn Asn Lys Met Tyr His Leu Ser Leu Gln Gly
    1400                1405                1410

Asn Glu Lys Thr Ile Cys Asn Asn Met Asn Ile Ile Gln Asn Asn
    1415                1420                1425

Ile Asn Lys Leu Phe Gly Ser Ser Asn Ile Lys Ser Ile Pro Tyr
    1430                1435                1440

Phe Tyr Lys Asn Asn Gly Leu Glu Asn Cys Ile Gly Val Phe Ser
    1445                1450                1455

Ile Lys Asn Asn Glu Phe Ile Tyr Met Leu Lys Tyr Asp Asp Lys
    1460                1465                1470

Asn Lys Ile Tyr Lys Tyr Lys Asp Asn Asn Leu Val Cys Gly Phe
    1475                1480                1485

Asn Leu Phe Asp Ile Ser Tyr Val Asp Ile Ile Ser Asn Gly Asp
    1490                1495                1500

Lys Asn Tyr Leu Lys Gly Ile Trp Asn Thr Ser Val Asn Asn Ser
    1505                1510                1515

Glu Tyr Lys Ile Glu Phe Leu Val Glu Leu Ile Asp Gln Asn Thr
    1520                1525                1530

Ile Glu Ile Ile Lys Leu Asn Leu Ser Asp Gly Leu Ile Lys Ser
    1535                1540                1545

Phe Leu Thr Ile Leu Asp Asn Leu Glu Ser Leu Asp Val Lys Ile
    1550                1555                1560

Ser Thr Ile Tyr Asp Phe Leu Lys Asn Ile Leu Phe Arg Asp Val
    1565                1570                1575

Phe Leu Glu Asn Glu Ser Leu Asn Phe Ile Val Ser Lys Gly Phe
    1580                1585                1590

Val Leu Thr Gly Asn Asn Ser Lys Asn Lys Tyr Glu Phe Ile Cys
    1595                1600                1605

Asn Ser Glu Asn Ile Gln Leu Tyr Ile Thr Glu Leu Tyr Thr Asn
    1610                1615                1620

Gly Thr Lys Phe Arg Asn Trp Leu Asp Asn Gly Lys Ile Leu Val
    1625                1630                1635

Ser Ser Thr Gly Glu Ser Ser Asn Asn Leu Ile Asp Ile
    1640                1645                1650

1